# EUROPÄISCHE PATENTSCHRIFT

(11) **EP 3 555 315 B1**
(45) Veröffentlichungstag und Bekanntmachung des Hinweises auf die Patenterteilung: **09.07.2025**
(21) Anmeldenummer: 17832048.7
(22) Anmeldetag: 06.12.2017
(51) Int. Cl.: C12Q 1/6844, B01L 7/00

(54) **VERFAHREN UND SYSTEM ZUM VERVIELFÄLTIGEN EINER NUKLEINSÄURE**
METHOD AND SYSTEM FOR MULTIPLY COPYING A NUCLEIC ACID
PROCÉDÉ ET SYSTÈME DE MULTIPLICATION D'UN ACIDE NUCLÉIQUE

(30) Priorität: 16.12.2016 DE 102016124692
(43) Veröffentlichungstag der Anmeldung: 23.10.2019
(73) Patentinhaber: HP Health Solutions Germany GmbH, 82166 Gräfelfing (DE)
(72) Erfinder: STEHR, Joachim, 82166 Gräfelfing (DE); STEMPLINGER, Ilse, 82166 Gräfelfing (DE); URBAN, Cordula, 82166 Gräfelfing (DE); ZIGANN, Katja, 82166 Gräfelfing (DE); VANESKI, Aleksander, 82166 Gräfelfing (DE); BUERSGENS, Federico, 82166 Gräfelfing (DE); ULLERICH, Lars, 82166 Gräfelfing (DE)
(74) Vertreter: Tautz & Schuhmacher
(86) Internationale Anmeldenummer: PCT/EP2017/081754
(87) Internationale Veröffentlichungsnummer: WO 2018/108680

(56) Entgegenhaltungen:
- WO-A1-2007/143034
- WO-A1-2013/113910
- WO-A1-99/22030
- US-A1- 2011 028 334

## Beschreibung

Die vorliegende Erfindung betrifft ein Verfahren und ein System zum Vervielfältigen einer Nukleinsäure. Ferner betrifft die Erfindung eine Primernukleinsäure, eine Primerkomplementärnukleinsäure, sowie ein Lokalheizelement für eine Polymerase-Kettenreaktion zum Vervielfältigen einer Nukleinsäure.

### Stand der Technik

Aus der DE 10 2012 201 475 A1 scheint ein Verfahren zum Vervielfältigen einer Nukleinsäure bekannt zu sein, bei welchem gemäß zumindest mancher Ausführungsformen Nanopartikel-Oligonukleotid-Konjugate verwendet werden. Dabei sind die Nanopartikel derart mit Primern verbunden, dass kovalente Bindungen mit zumindest einem Thiollinker zwischen Primern und Nanopartikeln vorhanden sind, um eine Gefahr eines Ablösens der Primer, insbesondere während eines Denaturierungsschrittes, zu verringern und eine Effizienz der PCR zu erhöhen.

Die US 2003/0022169 A1 scheint kein Verfahren zum Vervielfältigen von Nukleinsäure, hingegen ein Verfahren zum Detektieren von Nukleinsäuren zu offenbaren. Dazu wird die nachzuweisende Nukleinsäure scheinbar mit einer Art von Nanopartikeln verbunden, auf welchen Oligonukleotide angebracht sind, so dass diese Nanopartikel-Oligonukleotid-Konjugate bilden. Das Verfahren scheint darauf zu beruhen, dass eine Hybridisierung der an den Nanopartikeln angebrachten Oligonukleotide mit der zu detektierenden Nukleinsäure eine messbare Änderung hervorruft. Zudem scheint ein System zur Detektion einer bestimmten Nukleinsäure offenbart zu sein, bei welcher Oligonukleotide an den Nanopartikeln befestigt sind. Diese Oligonukleotide können dann wiederum mit einem Bindungsnukleotid hybridisieren, welches zumindest zwei Abschnitte aufweist, wobei ein erster Abschnitt zu zumindest einer Teilsequenz der am Nanopartikel angebrachten Oligonukleotide komplementär ist und ein zweiter Teil zu einer Teilsequenz der Nukleinsäure komplementär ist.

Die US 2004/038229 A1 scheint ein Verfahren zur enzymatischen Manipulation von an Metallpartikel gebundener DNA zu offenbaren. Insbesondere scheint offenbart zu sein, Nanopartikel mit einem direkt daran gebundenen einzelsträngigen DNA Primer bereitzustellen, eine einzelsträngige, zu vervielfältigende DNA mittels Annealing mit dem Primer zu verbinden. Nach erfolgter Vervielfältigung, welche unabhängig von dem Nanopartikel zu erfolgen scheint, scheint das Nanopartikel zur Detektion der amplifizierten Nukleinsäure verwendet zu werden.

Die US 2011/0274706 A1 scheint ein Vehikel zum Befördern von Nukleinsäuren zu Target-Zellen zu offenbaren, wobei das Vehikel eine Mehrzahl von Nanopartikeln und eine Mehrzahl von Nukleinsäuren umfasst. Die Nanopartikel und die Nukleinsäuren sind dabei derart agglomeriert, dass diese ein Nukleinsäuren-Granulations-Partikel mit einer Größe von zumindest 20 nm bilden. In diesem Zusammenhang scheint ferner offenbart zu sein, dass die Nukleinsäuren über Linker, welche als Oligonukleotide, wie etwa spezifische Primer, ausgebildet sein können an die Nanopartikel gebunden sein können.

Aus der EP 1 179 185 B1 scheint ein Verfahren zum Nachweis von Analyten mit Hilfe von Halbleiter Nanokristallen bekannt zu sein. Im Zusammenhang mit einer als "fluorescence in situ hybridization (FISH)" bezeichneten Methode zur Detektion von biologischen Proben scheint dabei offenbart zu sein, dass DNA Primer über Nanokristall-verbundene Nukleotide mit Nanokristallen verbunden werden können. Unabhängig von dem Nachweisverfahren scheint eine herkömmliche PCR verwendet werden zu können, um Nukleinsäure-Fragmente für die FISH Proben zu erzeugen.

Aus der US 2016/6265044 A1 scheinen Konjugate offenbart zu sein, bei welchen ein Biomolekül mit einem Label verbunden ist, insbesondere mit Polymerasen, wobei das Konjugat Polymerase-Aktivität aufweist. Ferner scheint allgemein offenbart zu sein, dass die Biomoleküle und/oder Labels an eine Oberfläche gebunden sind, wobei die Bindung zu einer reversiblen oder irreversiblen Immobilisierung von Nanopartikeln, Polymerasen, Oligonukleotiden und Primern etc. an einer Oberfläche führen kann. Wie ferner offenbart zu sein scheint, können geeignete Linker zum Verbinden von Biomolekülen, Labels, wie etwa Nanopartikel, und einer Oberfläche verwendet werden. Die Linker können demnach scheinbar via kovalenter Bindung, nicht-kovalenter Bindung, ionischer Bindung, hydrophobischer Wechselwirkungen oder eine Kombination daraus an den Oberflächen, den Nanopartikeln und/oder den Primern befestigt sein.

Aus der US 2014/0170664 A1 scheint ein Heizmechanismus für DNA-Anwendungen bekannt zu sein. Insbesondere scheint gemäß einer bevorzugten Ausführungsform offenbart zu sein, eine PCR-Lösung durch das Bestrahlen von darin befindlichen Nanopartikel zu beheizen. Bevorzugt scheinen dabei Gold-Nanokugeln verwendet zu werden, welche mittels einer kovalenten Bindung, insbesondere mittels einer Thiol-Bindung, fest und irreversibel an Primer für die PCR gebunden sind.

Aus der US 2014/0127695 A1 scheint ein Verfahren zur Bestimmung von Nukleinsäure bekannt zu sein, bei welchem magnetische Nanopartikel und detektierbare Nanopartikel bereitgestellt werden. Ferner scheint dabei gemäß einer Ausführungsform ein Nanopartikel-basiertes System zur Amplifikation einer Nukleinsäure, beispielsweise mittels PCR, bereitgestellt zu werden. An den Nanopartikeln sind dabei Primer befestigt. Nachdem die auf den Nanopartikeln angebrachten Primer elongiert wurden, können diese zu einem Komplex hybridisieren. Darüber hinaus scheint offenbart zu sein, dass zu Detektionszwecken PCR Primer durch an Nanopartikel beschichtete Primer ersetzt werden.

Aus der EP 2 110 175 A1 scheint ein Verfahren zur thermischen Steuerung mindestens einer temperaturabhängigen Hybridisierungs- oder Bindungsreaktion oder einer enzymatischen Reaktion bekannt zu sein, um eine Amplifizierung in Gegenwart magnetischer Nanopartikel durchzuführen. Insbesondere scheint daraus bekannt zu sein, einzelsträngige Nukleinsäuren, wie etwa Oligonukleotide oder PCR-Produkte, über Linkermoleküle an eine Bead-Oberfläche zu binden.

Aus der Veröffentlichung Liu et al., Biotechnol. J. 2007, 2, 508-511: "PCR amplification on magnetic nanoparticles: Application for high-throuput single nucleotide polymorphism genotyping" scheint ein Verfahren bekannt zu sein, bei welchem PCR Produkte direkt auf magnetischen Nanopartikeln amplifiziert werden, wobei Reverse-Primer mittels einer kovalenten Bindung auf den magnetischen Nanopartikeln immobilisiert zu sein scheinen.

Aus der US 6,953,659 B2 scheint eine Methode bekannt zu sein, bei welcher Modulatoren, welche als Metall Nanopartikel ausgebildet sein können, verwendet werden, um Wärme zu einer Nukleinsäure zu transferieren, wobei die Nukleinsäure direkt an einen der Modulatoren gebunden ist. Darüber hinaus scheinen die Modulatoren zum lokalen Heizen in einer PCR verwendet zu werden. Ferner scheint offenbart zu sein, dass Primer für eine Amplifikationsreaktion derart modifiziert sind, dass diese einen Modulator aufweisen.

Die WO2004/055160 A2 offenbart eine Synthese von fluoreszenter, einzelsträngiger DNA unter Verwendung von "Klick-Chemie", sowie die Verwendung der fluoreszenten einzelsträngigen DNA als ein Primer zur Herstellung von DNA-Sequenzierungsfragmenten. Mittels "Klick-Chemie" werden Biomoleküle an andere Komponenten gebunden, beispielsweise etwa wird DNA mittels kovalenter Bindung an einen Chip gebunden.

Die WO2016/074701A1 offenbart Nanopartikel, wobei einstückig ausgebildete Primernukleinsäuren direkt an die Nanopartikel gebunden sind. Die einstückig ausgebildeten Primernukleinsäuren weisen zwei Teilsequenzen auf, wovon eine Teilsequenz als eine Primersequenz ausgebildet ist und die andere Teilsequenz als eine Spacersequenz ausgebildet ist, mittels welcher die Primersequenz vom Nanopartikel beabstandet wird. Die beiden Teilsequenzen der einstückigen Primernukleinsäuren können optional über eine abasische Modifikation miteinander verbunden sein.

Die WO 99/22030 A1 offenbart ein Verfahren zur Sequenzierung mittels Durchflusszytometrie, bei welchem Primer an Mikrokügelchen gebunden werden, deren Fluoreszenz zur Detektion verwendet wird.

Die WO 2013/113910 A1 offenbart ein Verfahren zur Amplifikation von Nukleinsäuren, bei welchem Primer direkt an ein Nanopartikel gebunden sein können.

### Der Erfindung zugrunde liegende Aufgabe

Der Erfindung liegt die Aufgabe zugrunde, ein einfacher bereitstellbares und/oder flexibleres System und ein einfacher durchführbares Verfahren zum Vervielfältigen einer Nukleinsäure bereitzustellen. Darüber hinaus liegt der Erfindung die Aufgabe zugrunde, Komponenten für ein einfacher bereitstellbares bzw. flexibleres System und ein einfacher durchführbares Verfahren zum Vervielfältigen einer Nukleinsäure bereitzustellen.

### Offenbarung der Erfindung

Die Aufgabe wird gelöst durch ein erfindungsgemäßes Verfahren, ein erfindungsgemäßes System, sowie ein erfindungsgemäßes Lokalheizelement mit den Merkmalen der entsprechenden unabhängigen Ansprüche. Bevorzugte Ausführungsformen ergeben sich aus den abhängigen Ansprüchen, sowie aus der Beschreibung und den Figuren.

In einem ersten Aspekt betrifft die Erfindung ein System zum Vervielfältigen einer Nukleinsäure, umfassend zumindest ein Lokalheizelement, welches mit zumindest einer Verbindungsnukleinsäure funktionalisiert ist, und zumindest eine Primernukleinsäure, welche dazu ausgelegt ist, an die zumindest eine Verbindungsnukleinsäure zu binden und an die Nukleinsäure zu binden. Alternativ oder zusätzlich zur Primernukleinsäure kann das System zumindest eine Primerkomplementärnukleinsäure umfassen, welche dazu ausgelegt ist, an die zumindest eine Verbindungsnukleinsäure zu binden, und die Verbindungsnukleinsäure mittels einer enzymatischen Reaktion um eine Primernukleotidsequenz zu elongieren. In einem weiteren Aspekt betrifft die Erfindung ein Verfahren zum Vervielfältigen einer Nukleinsäure in einer Reaktionslösung, umfassend ein Bereitstellen von zumindest einem Lokalheizelement in der Reaktionslösung, wobei das Lokalheizelement mit zumindest einer Verbindungsnukleinsäure funktionalisiert ist. Darüber hinaus umfasst das Verfahren ein Bereitstellen und/oder Erzeugen von zumindest einer Primernukleinsäure in der Reaktionslösung, wobei die Primernukleinsäure dazu ausgelegt ist, an die zumindest eine Verbindungsnukleinsäure zu binden und an die Nukleinsäure zu binden. Ferner umfasst das Verfahren ein Übertragen von Wärme durch das Lokalheizelement an eine Umgebung des Lokalheizelements derart, dass eine über die zumindest eine Primernukleinsäure und die zumindest eine Verbindungsnukleinsäure mit dem zumindest einen Lokalheizelement verbundene Nukleinsäure auf und/oder über eine Denaturierungstemperatur erhitzt wird.

In einem weiteren Aspekt betrifft die Erfindung eine Primernukleinsäure für eine Polymerase-Kettenreaktion zum Vervielfältigen einer Nukleinsäure, umfassend einen Verbindungsabschnitt und einen Primerabschnitt, wobei der Verbindungsabschnitt dazu ausgelegt ist, an eine Verbindungsnukleinsäure zu binden, und wobei der Primerabschnitt dazu ausgelegt ist, zumindest teilweise an die Nukleinsäure zu binden und in der Polymerase-Kettenreaktion als Primer zu agieren.

In einem weiteren Aspekt betrifft die Erfindung die Verwendung einer Primernukleinsäure als Primer für eine Polymerase-Kettenreaktion zum Vervielfältigen einer Nukleinsäure, wobei die Primernukleinsäure einen Verbindungsabschnitt und einen Primerabschnitt umfasst, wobei der Verbindungsabschnitt dazu ausgelegt ist, an eine Verbindungsnukleinsäure zu binden, und wobei der Primerabschnitt dazu ausgelegt ist, zumindest teilweise an die Nukleinsäure zu binden und in der Polymerase-Kettenreaktion als Primer zu agieren.

In einem weiteren Aspekt betrifft die Erfindung eine Primerkomplementärnukleinsäure für eine Polymerase-Kettenreaktion zum Vervielfältigen einer Nukleinsäure, umfassend einen Verbindungsabschnitt und einen Primerkomplementärabschnitt, wobei der Verbindungsabschnitt dazu ausgelegt ist, an eine Verbindungsnukleinsäure zu binden, und wobei der Primerkomplementärabschnitt dazu ausgelegt ist, die Verbindungsnukleinsäure mittels einer enzymatischen Reaktion um eine Primernukleotidsequenz und/oder einer Primernukleinsäure zu elongieren.

In einem weiteren Aspekt betrifft die Erfindung die Verwendung einer Primerkomplementärnukleinsäure als eine Primervorlage für eine Polymerase-Kettenreaktion zum Vervielfältigen einer Nukleinsäure, umfassend einen Verbindungsabschnitt und einen Primerkomplementärabschnitt, wobei der Verbindungsabschnitt dazu ausgelegt ist, an eine Verbindungsnukleinsäure zu binden, und wobei der Primerkomplementärabschnitt dazu ausgelegt ist, die Verbindungsnukleinsäure mittels einer enzymatischen Reaktion um eine Primernukleotidsequenz und/oder einer Primernukleinsäure zu elongieren.

In einem weiteren Aspekt betrifft die Erfindung ein Lokalheizelement für eine Polymerase-Kettenreaktion zum Vervielfältigen einer Nukleinsäure, wobei das Lokalheizelement mit zumindest einer Verbindungsnukleinsäure funktionalisiert ist und die zumindest eine Verbindungsnukleinsäure dazu ausgelegt ist, an eine Primernukleinsäure und/oder an eine Primerkomplementärnukleinsäure zu binden.

In einem weiteren Aspekt betrifft die Erfindung die Verwendung eines Lokalheizelements in einer Polymerase-Kettenreaktion zum Vervielfältigen einer Nukleinsäure, wobei das Lokalheizelement mit zumindest einer Verbindungsnukleinsäure funktionalisiert ist und die zumindest eine Verbindungsnukleinsäure dazu ausgelegt ist, an eine Primernukleinsäure und/oder an eine Primerkomplementärnukleinsäure zu binden.

Ein Vervielfältigen einer Nukleinsäure ist dabei insbesondere ein Amplifizieren einer Nukleinsäure, vorzugsweise mittels einer enzymatischen Reaktion. Mit anderen Worten entspricht das Vervielfältigen einer Nukleinsäure vorzugsweise im Wesentlichen dem Duplizieren einer oder mehrerer Nukleinsäuren, insbesondere spezifischer Nukleinsäuren, d.h. von Nukleinsäuren, deren Nukleotidsequenz zumindest teilweise bekannt ist und welche anhand der bekannten Nukleotidsequenz vorzugsweise spezifisch ausgewählt und/oder vervielfältigt wird. Mit anderen Worten betrifft das Vervielfältigen einer Nukleinsäure vorzugsweise ein Kopieren der Nukleinsäuren, d.h. das Erstellen von im Wesentlichen identischen Amplikons bzw. Kopien der zu vervielfältigenden Nukleinsäure.

Das Vervielfältigen der Nukleinsäure erfolgt bevorzugt mittels einer Polymerase-Kettenreaktion, welche im Folgenden auch als PCR (engl.: Polymerase-Chain-Reaction) bezeichnet wird. Eine PCR im Sinne der vorliegenden Erfindung ist ein Verfahren zum Vervielfältigen von Nukleinsäuren, bei dem ein Vervielfältigungszyklus bestehend aus den Schritten Denaturierung, Hybridisierung und Elongation wiederholt durchlaufen wird, und zwar vorzugsweise in dieser Reihenfolge. In jedem Durchlauf kann die Anzahl der Nukleinsäuremoleküle vergrößert (im typischerweise besten Fall verdoppelt) werden, sodass es zu einer exponentiellen Zunahme der Zahl von Nukleinsäuremolekülen kommen kann. Im Folgenden wird eine zu vervielfältigende Nukleinsäure als "Original" bezeichnet. Das Original ist ein Einzelstrang und kann zusammen mit seinem komplementären Strang, der als "Komplement" bezeichnet wird, einen Doppelstrang bilden. Das Original und auch das Komplement können Teil einer größeren Nukleinsäure sein. Insbesondere kann bei einer PCR eine in einem Durchlauf des Vervielfältigungszyklus entstandene Kopie des Originals eine Vorlage zum Bilden eines Komplements in einem folgenden Durchlauf und eine entstandene Kopie des Komplements eine Vorlage zur Bildung eines Originals in einem folgenden Durchlauf sein. Eine geläufige Bezeichnung für das Amplifikationsprodukt ist "Amplikon".

Der Denaturierungsschritt dient dazu, einen Nukleinsäure-Doppelstrang zu denaturieren, das heißt, ihn in seine beiden Einzelstränge aufzutrennen. So kann in dem Denaturierungsschritt zum Beispiel das Original von dem Komplement getrennt werden. Die erfindungsgemäß bevorzugte Art der Denaturierung ist eine thermische Denaturierung (auch als "Schmelzen" bezeichnet). Dazu wird zumindest ein Teil des Nukleinsäure-Doppelstrangs oder der ganze Doppelstrang einer Temperatur, als "Denaturierungstemperatur" bezeichnet, ausgesetzt, die ein Trennen der Nukleinsäure-Doppelstränge hervorruft oder zumindest begünstigt. Die bevorzugte Denaturierungstemperatur ist einerseits so hoch gewählt, dass Nukleinsäure-Doppelstränge aufgetrennt werden können. Andererseits kann die bevorzugte Denaturierungstemperatur so niedrig gewählt werden, dass eine DNA-Polymerase, die sich möglicherweise ebenfalls in der Probe befindet, und sich gegebenenfalls ebenfalls in dem geheizten Bereich bzw. in der Umgebung eines Lokalheizelements befindet, nicht wesentlich geschädigt wird. Da eine Wahrscheinlichkeit, mit welcher sich eine Polymerase in der Umgebung eines Lokalheizelements aufhält jedoch sehr gering ist, kann eine Berücksichtigung der Polymerase bei der Wahl der Denaturierungstemperatur entbehrlich sein. Ein typischer Wert für die Denaturierungstemperatur ist 95°C.

Zur Erleichterung der nachfolgenden Erläuterung der Erfindung bezeichnet "Denaturierungsschritt" in der Nomenklatur der vorliegenden Erfindung den Schritt des Verfahrens, in dem die Heizeinrichtung Wärme erzeugt, um zumindest einen Teil des Reaktionsvolumens zu erwärmen bzw. zu erhitzen und dadurch eine Denaturierung doppelsträngiger Nukleinsäuremoleküle in dem erhitzten Teil des Reaktionsvolumens zu bewirken. Die Dauer des Denaturierungsschritts ist demnach die Summe der Zeit, in der die Heizeinrichtung in dem den Denaturierungsschritt betreffenden Durchlauf des Zyklus der PCR Wärme erzeugt. Im Fall eines Heizwiderstands als Heizeinrichtung ist die Dauer des Denaturierungsschritts also vorzugsweise die gesamte Dauer einer Stromdurchleitung durch die Heizeinrichtung, um das Reaktionsvolumen zu erwärmen und dadurch eine Denaturierung doppelsträngiger Nukleinsäuremoleküle zu bewirken. Im Fall optothermisch angeregter Nanopartikel als Lokalheizelement bzw. als Heizeinrichtung ist die Dauer des Denaturierungsschritts also die Dauer, für die die optische Anregungsquelle das Nanopartikel trifft, um das Reaktionsvolumen zumindest teilweise zu erwärmen und dadurch eine Denaturierung doppelsträngiger Nukleinsäuremoleküle zu bewirken. Wenn die Heizeinrichtung in einem Durchlauf des Vervielfältigungszyklus die Wärme statt in einem in mehreren voneinander getrennten Zeitintervallen erzeugt, ist die Dauer des Denaturierungsschritts die Summe der Dauern dieser Intervalle. In dem so definierten Denaturierungsschritt ist insbesondere nicht eine Abgabe von Wärme aufgrund der der Heizeinrichtung eigenen Wärmekapazität eingeschlossen, und auch nicht das Abklingen der Temperatur in dem an die Heizeinrichtung angrenzenden Teil des Reaktionsvolumens, selbst wenn die dort vorhandenen Temperaturen noch innerhalb des für eine Denaturierung erforderlichen Bereichs liegen. Dies bedeutet insbesondere, dass in dem erfindungsgemäßen Verfahren auch nach dem so definierten Denaturierungsschritt noch Denaturierung stattfinden kann. Es bedeutet auch, dass die im Denaturierungsschritt abgegebene Wärme in der Regel geringer ist als die im Denaturierungsschritt erzeugte Wärme.

Die PCR nutzt ferner vorzugsweise mindestens zwei Oligonukleotide, die als "Primer" bezeichnet werden, einen Vorwärtsprimer (auch als "Forward Primer" bezeichnet) und einen Rückwärtsprimer (auch als "Reverse Primer" bezeichnet). Der Vorwärtsprimer ist komplementär zum 3'-Ende des Originals und der Rückwärtsprimer ist komplementär zum 3'-Ende des Komplements. Im Hybridisierungsschritt (auch als "Annealingschritt" bezeichnet) hybridisiert der Vorwärtsprimer und/oder der Rückwärtsprimer an eine ihm komplementäre Sequenz im Original bzw. Komplement bzw. Amplikon. Der Hybridisierungsschritt findet gewöhnlich bei einer Temperatur statt, die ein Hybridisieren der Vorwärts- und Rückwärtsprimer an deren komplementäre Sequenzen im Original bzw. Komplement bzw. Amplikon hervorruft oder zumindest begünstigt. Sie wird vorzugsweise so gewählt, dass sie ein möglichst spezifisches Hybridisieren der Primer ermöglicht. Die Hybridisierungstemperatur liegt typischerweise zwischen 50° C und 72°C.

Im Elongationsschritt werden die hybridisierten Primer von einem Polymerase-Enzym komplementär verlängert bzw. elongiert. So kann ausgehend von dem Vorwärtsprimer ein Komplement und ausgehend von dem Rückwärtsprimer ein Original synthetisiert werden. Zum Zweck der Elongation wird die Polymerase einer Temperatur ausgesetzt, die eine Elongation ermöglicht oder zumindest begünstigt. Bei der Verwendung einer Polymerase von dem Bakterium Thermus aquaticus (Taq) wird typischerweise eine Elongationstemperatur von 72 ° C verwendet. In manc hen Ausführungsformen der PCR sind die Hybridisierungs- und die Elongationstemperaturen identisch, das heißt, beide Schritte finden bei der gleichen Temperatur statt (das heißt, es gibt nur zwei Temperaturstufen während der PCR, eine kombinierte Hybridisierungs- und Elongationstemperatur und eine Denaturierungstemperatur).

Die Bezeichnungen "Nukleinsäure" und "Oligonukleotid" umfassen im Zusammenhang mit der vorliegenden Erfindung nicht nur (Desoxy)-Ribonukleinsäuren bzw. (Desoxy)-Oligo-Ribonukleotide, auch wenn vorgenannte bevorzugt sind, sondern auch Nukleinsäuren und Oligonukleotide, die ein oder mehrere Nukleotid-Analoga mit Modifikationen an ihrem Backbone (zum Beispiel Methylphosphonate, Phosphothioate oder Peptide Nucleic Acids (PNA), insbesondere an einem Zucker des Backbone (zum Beispiel 2'-O-Alkylderivate, 3'- und/oder 5'-Aminoribosen, Locked Nucleic Acids [LNA], Hexitol Nucleic Acids, Morpholinos, Glycol Nucleic Acid (GNA), Threose Nucleic Acid (TNA) oder Tricyclo-DNA, vergleiche hierzu den Aufsatz von D. Renneberg und C.J. Leumann, "Watson-Crick base-pairing properties of Tricyclo-DNA", J. Am. Chem. Soc., 2002, Bd. 124, Seiten 5993-6002, dessen diesbezüglicher Inhalt durch Verweis Teil der vorliegenden Offenbarung ist) enthalten oder die Basen-Analoga enthalten, zum Beispiel 7-Deazapurine oder Universalbasen wie Nitroindol oder modifizierte natürliche Basen wie N4-Ethyl-Cytosin. In einer Ausführung der Erfindung sind die Nukleinsäuren oder Oligonukleotide Konjugate oder Chimären mit nichtnukleosidischen Analoga, zum Beispiel PNA. Die Nukleinsäuren oder Oligonukleotide enthalten in einer Ausführung der Erfindung an einer oder mehreren Positionen nichtnukleosidische Einheiten wie Spacer, zum Beispiel Hexaethylenglycol oder Cn-Spacer mit n zwischen 3 und 6. Soweit die Nukleinsäuren oder Oligonukleotide Modifikationen enthalten, sind diese so gewählt, dass auch mit der Modifikation eine Hybridisierung mit natürlichen DNA/RNA-Analyten möglich ist. Bevorzugte Modifikationen beeinflussen das Schmelzverhalten, vorzugsweise die Schmelztemperatur, insbesondere um Hybride mit unterschiedlichen Graden der Komplementarität ihrer Basen unterscheiden zu können (Mismatch-Diskriminierung). Bevorzugte Modifikationen umfassen LNA, 8-Aza-7-Deaza-Purine, 5-Propinyl-Uracil und -Cytosin und/oder abasische Unterbrechungen oder Modifikationen in der Nukleinsäure oder in dem Oligonukleotid. Weitere Modifikationen im Sinne der Erfindung sind zum Beispiel Modifikationen mit Biotin, Thiol und Fluoreszenzdonor- und Fluoreszenzakzeptormolekülen.

Abasische Modifikationen sind vorzugsweise aus einer Gruppe ausgewählt, die umfasst: 1', 2'-Dideoxyribose (dSpacer), Triethylen-Glycol (Spacer9) und HexaEthylenglycol (Spacer18).

Das erfindungsgemäße Verfahren läuft in einem Reaktionsvolumen bzw. in einer Reaktionslösung ab. Dies bedeutet im Sinne der vorliegenden Erfindung, dass die Vervielfältigung der Nukleinsäuren zumindest in einem Teil eines zusammenhängenden Reaktionsvolumens stattfindet. Das Reaktionsvolumen ist eine flüssige Lösung bzw. zumindest ein Teil einer Reaktionslösung oder Suspension, die neben dem Lösungs- oder Suspensionsmittel, vorzugsweise Wasser, für gewöhnlich auch die zu vervielfältigende(n) Nukleinsäure(n) (im Folgenden auch als "Target" oder "Target-Nukleinsäure(n)" genannt) enthält. Es enthält außerdem in der Regel Originale und Komplemente und/oder andere Bestandteile, zum Beispiel Polymerase(n), dNTPs und Salze, die suspendiert oder gelöst sein können.

Eine Verbindungsnukleinsäure ist dabei insbesondere eine Nukleinsäure, welche an ein Lokalheizelement funktionalisiert ist und eine Nukleotidsequenz aufweist, welche ein Binden bzw. Hybridisieren einer anderen Nukleinsäure, insbesondere einer Primernukleinsäure und/oder einer Primerkomplementärnukleinsäure, an die Verbindungsnukleinsäure ermöglicht. Vorzugsweise liegt die Verbindungsnukleinsäure als eine einzelsträngige Nukleinsäure, insbesondere als ein einzelsträngiges Oligonukleotid, vor. Bevorzugt fungiert die Verbindungsnukleinsäure nicht als Primer für die PCR, d.h. die Verbindungsnukleinsäure weist bevorzugt keine Nukleotidsequenz auf, welche in einer PCR als Primer fungiert. Bevorzugt weist die Verbindungsnukleinsäure eine möglichst universelle Nukleotidsequenz auf, welche es dem Benutzer ermöglicht, andere Nukleinsäuren, welche zum Binden an die Verbindungsnukleinsäure vorgesehen sind, auf einfache Weise anzupassen bzw. in geeigneter Weise auszugestalten. Dies bedeutet, dass die zum Binden an die Verbindungsnukleinsäure vorgesehene Nukleinsäure zumindest teilweise mit einer Nukleotidsequenz versehen werden kann, welche, beispielsweise in einem Überhang, zu der möglichst universellen Nukleotidsequenz der Verbindungsnukleinsäure zumindest teilweise komplementär ist.

Eine Primernukleinsäure ist dabei eine Nukleinsäure, welche zum Vervielfältigen der Nukleinsäure als Primer dient bzw. dienen kann. Vorzugsweise liegt die Primernukleinsäure als ein Oligonukleotid vor. Besonders bevorzugt ist dabei zumindest ein Teil der Nukleotidsequenz der Primernukleinsäure zumindest teilweise komplementär zu der zu vervielfältigenden Nukleinsäure. Die Primernukleinsäure kann dabei beispielsweise als ein Vorwärtsprimer und/oder als ein Rückwärtsprimer ausgebildet sein. Die Primernukleinsäure kann insbesondere mehrere Teile aufweisen, wobei beispielsweise ein Teil dazu ausgelegt ist, als ein Primer für die PCR zu agieren, während ein anderer Teil oder mehrere andere Teile mit anderen Funktionen bedacht sind. Beispielsweise kann ein anderer Teil der Primernukleinsäure dazu ausgelegt sein, an die Verbindungsnukleinsäure zu binden.

Eine Primerkomplementärnukleinsäure ist dabei eine Nukleinsäure, welche zur Erzeugung von Primern dient. Vorzugsweise liegt die Primerkomplementärnukleinsäure als ein Oligonukleotid vor. Bevorzugt ist dabei zumindest ein Teil der Nukleotidsequenz der Primerkomplementärnukleinsäure zumindest teilweise komplementär zu einem Primer zur Vervielfältigung der Nukleinsäure. Mit anderen Worten ist die Primerkomplementärnukleinsäure vorzugsweise zumindest teilweise derart ausgestaltet, das beispielsweise durch eine enzymatische Reaktion zumindest ein Teil der Primerkomplementärnukleinsäure derart zu einem Doppelstrang komplettiert werden kann, dass der dabei entstehende Abschnitt in einem weiteren Schritt als ein Primer zum Vervielfältigen der Nukleinsäure verwendet werden kann. Mit anderen Worten dient vorzugsweise zumindest ein Teil der Primerkomplementärnukleinsäure als eine Vorlage zur Erzeugung von Primern. Ferner kann die Primerkomplementärnukleinsäure vorzugsweise mehrere Teile aufweisen, wobei beispielsweise ein Teil dazu ausgelegt ist, als eine Vorlage für die Erzeugung von Primern zu fungieren, während ein anderer Teil oder mehrere andere Teile der Primerkomplementärnukleinsäure mit anderen Funktionen bedacht sein können. Beispielsweise kann ein anderer Teil der Primerkomplementärnukleinsäure dazu ausgelegt sein, an die Verbindungsnukleinsäure zu binden, so dass vorzugsweise beim Komplettieren der Primerkomplementärnukleinsäure die an einen anderen Teil der Primerkomplementärnukleinsäure gebundene Verbindungsnukleinsäure elongiert werden kann.

Zum Komplettieren der Primerkomplementärnukleinsäure bzw. zum Elongieren der Verbindungsnukleinsäure kann beispielsweise ein Enzym bzw. das gleiche bzw. das selbe Enzym, wie etwa eine Polymerase dienen, welches auch zum Elongieren von Primern bei der Vervielfältigung bzw. Amplifizierung der Nukleinsäure in der Reaktionslösung, insbesondere im Rahmen einer PCR, dient. Alternativ oder zusätzlich können zumindest ein weiteres Enzym bzw. zumindest eine weitere Polymerase bzw. Art von Polymerasen in der Reaktionslösung bereitgestellt werden, welche zum Komplettieren der Primerkomplementärnukleinsäure dienen, jedoch von der Polymerase zum Elongieren der Primer verschieden ist. In diesem Fall würden in der Reaktionslösung vorzugsweise zumindest zwei Arten von Polymerasen vorliegen. Gemäß einer bevorzugten Ausführungsform kann beispielsweise ein Enzym, welches lediglich zum Elongieren der Verbindungsnukleinsäure verwendet wird, nach erfolgtem Elongieren der Verbindungsnukleinsäure und zeitlich vor dem Vervielfältigen bzw. Amplifizieren der Nukleinsäure, zumindest teilweise aus der Reaktionslösung entfernt, wie etwa herausgewaschen, werden.

Ein Lokalheizelement im Sinne dieser Erfindung ist ein Heizelement, welches insbesondere dazu ausgelegt ist, durch eine in bzw. von dem Lokalheizelement bereitgestellte Wärmeenergie lediglich ein räumlich begrenztes Teilvolumen des Reaktionsvolumens bzw. der Reaktionslösung um das Lokalheizelement herum zu erhitzen. Bevorzugt nimmt dazu ein Volumen des Lokalheizelements bzw. der mehreren Lokalheizelemente lediglich einen kleinen Bruchteil des Gesamtvolumens des Reaktionsvolumens bzw. der Reaktionslösung ein, sodass das erhitzte Volumen nur einen kleinen Bruchteil des gesamten Reaktionsvolumens ausmacht. Dadurch werden insbesondere besonders schnelle Temperaturänderungen ermöglicht, da insbesondere eine thermische Trägheit des Systems bzw. des Lokalheizelements möglichst gering gehalten wird. Zum anderen ist durch die Verwendung eines Lokalheizelements oder mehrerer Lokalheizelemente eine sehr schnelle Abkühlung des erwärmten Volumens möglich, wenn ein ausreichend großes, kaltes TemperaturReservoir im Reaktionsvolumen vorhanden ist, wobei das kalte Temperaturreservoir eine kältere Temperatur aufweist, als die Temperatur der Umgebung eines beheizten Lokalheizelements, um nach dem Heizen das eine oder die mehreren Lokalheizelemente und ihre Umgebung wieder abzukühlen. Vorzugsweise entspricht die Temperatur des kalten Temperaturreservoirs im Wesentlichen der kombinierten Annealingtemperatur und/oder Elongationstemperatur ist. Dies kann dadurch erreicht werden, dass die Lokalheizelemente hinreichend stark (um den gewünschten Temperaturhub zu erreichen) und hinreichend kurz (damit die Wärme lokalisiert bleibt) beheizt werden. Auf diese Weise können mittels sehr schneller Temperaturänderungen, welche jeweils in einem sehr begrenzten räumlichen Teilvolumen des Reaktionsvolumens um ein Lokalheizelement herum stattfinden, Temperaturzyklen für die Vervielfältigungsreaktion von sehr kurzer Zeitdauer und in sehr kurzen Zeitabständen erzielt werden. Ein Lokalisieren des Erwärmens bzw. Erhitzens der Reaktionslösung in einer Umgebung bzw. Umgebungen von einem oder mehreren Lokalheizelementen hat dabei zur Folge, dass auch eine Denaturierung von Nukleinsäuren in der Umgebung bzw. den Umgebungen um das Lokalheizelement bzw. die Lokalheizelemente erfolgt. Aus diesem Grund kann es besonders vorteilhaft sein, zumindest einen Primer an das zumindest eine Lokalheizelement zu binden, insbesondere über eine Verbindungsnukleinsäure, um ein Denaturieren des Primers und des dabei entstehenden doppelsträngigen Amplikons in einzelsträngige Nukleinsäuren zu ermöglichen.

In einem weiteren Aspekt der Erfindung ist das Lokalheizelement so ausgelegt und wird so betrieben, dass die im Lokalheizelement erzeugte Wärme/Wärmemenge, die dem Reaktionsvolumen im Denaturierungsschritt zugeführt wird, weniger als C_{R} * 5° C (Grad Celsius) beträgt. Hierbei ist C_{R} die Wärmekapazität des Reaktionsvolumens während des Erwärmens durch das Lokalheizelement. Mit anderen Worten, bei Nichtberücksichtigung anderer Wärmezu und -abflüsse, erwärmt das Lokalheizelement das Reaktionsvolumen (über das Volumen gemittelt) um weniger als 5° C; d.h. sobald sich die Wärme im Reaktionsvolumen ausgebreitet hat (d.h. das Probenvolumen thermalisiert ist), beträgt die globale, durch das Lokalheizelement im Denaturierungsschritt eingebrachte, Temperaturerhöhung im gesamten Probenvolumen weniger als 5° C. Mit anderen Worten muss die Lokalheizenrichtung bzw. das Lokalheizelement dem Reaktionsvolumen im Denaturierungsschritt weniger Wärme zufügen, als benötigt würde, um das gesamte Reaktionsvolumen um 5°C zu erwärmen. Durch diesen geringen globalen Energie-Eintrag in das Reaktionsvolumen wird erreicht, dass sehr schnelle Temperaturzyklen möglich sind, da nur eine geringe Wärmemenge/Energiemenge dem Reaktionsvolumen nach dem Deanturierungsschritt zur Rückkehr auf die Annealing- und Elongationstemperatur entzogen werden muss, bzw. vorzugsweise sind die vom Lokalheizelement während des Denaturierungsschritts eingebrachten Energie-/Wärmemengen so gering, dass keine für die PCR signifikante globale Temperaturerhöhung eintritt und die Wärme dem Reaktionsvolumen nicht entzogen werden muss.

Die Erfindung bietet dabei den Vorteil, dass insbesondere im Vergleich zu herkömmlichen Methoden zur Vervielfältigung von Nukleinsäure mittels globalem Erhitzen der Reaktionslösung ein sehr schnelles Vervielfältigen der Nukleinsäure erreicht werden kann, da die erforderliche Zeitdauer für einen Zyklus der Vervielfältigungsreaktion, sowie die Zeitdauer zwischen zwei aufeinanderfolgenden Zyklen sehr viel kürzer ist, als bei herkömmlichen Verfahren, welche eines globalen Erhitzens der Reaktionslösung bedürfen.

Insbesondere bietet die Erfindung auch gegenüber dem aus der Druckschrift DE 10 2012 201 475 A1 bekannten LASER-PCR Verfahren den Vorteil, dass im Gegensatz dazu nicht für jede spezifische Anwendung, d.h. für das vervielfältigen einer jeden spezifischen Nukleinsäure, die Lokalheizelemente separat bzw. spezifisch funktionalisiert werden müssen. Mit anderen Worten müssen gemäß der Erfindung nicht für die Vervielfältigung einer jeden spezifischen Nukleinsäure eigens in dazu geeigneter Weise funktionalisierte Nanopartikel hergestellt werden, sondern vielmehr ermöglicht die Erfindung, dass durch ein Bereitstellen von Lokalheizelementen, welche mit einer universell verwendbaren Verbindungsnukleinsäure funktionalisiert sind, und ein Bereitstellen von für die zu vervielfältigen Nukleinsäure spezifisch angepasste Primernukleinsäuren und/oder Primerkomplementärnukleinsäuren, der Aufwand und/oder die Kosten und/oder die Herstellungsdauer reduziert werden können.

Ferner haben die Erfinder als Vorteil erkannt, dass ein optional auftretendes Auftrennen der Bindung von Primernukleinsäuren an die Verbindungsnukleinsäuren bzw. an die Lokalheizelemente während eines Denaturierungsschrittes nicht notwendigerweise nachteilbehaftet für das Vervielfältigen der Nukleinsäure sein muss, sondern dass dies für manche Ausführungsformen der Erfindung in vorteilhafter Weise genutzt werden kann. Folglich haben die Erfinder erkannt, dass nicht notwendiger Weise eine Bindung zwischen der Primernukleinsäure und dem Lokalheizelement erforderlich ist, welche auch in oder über einen Denaturierungsschritt hinaus zwingend erhalten bleibt.

Bevorzugt können dabei eine oder mehrere Verbindungsnukleinsäuren derart an das Lokalheizelement bzw. an jedes der Lokalheizelemente funktionalisiert werden, dass die Funktionalisierung im Wesentlichen irreversibel ist. Erfindungsgemäß kann insbesondere eine spezifische Anpassung des Systems bzw. des Verfahrens zum Vervielfältigen der spezifischen, gewünschten Nukleinsäure dadurch erfolgen, dass separat von dem einen oder mehreren Lokalheizelementen, welche mit universellen Verbindungsnukleinsäuren funktionalisiert sind, spezifische, an die zu amplifizierende Nukleinsäure angepasste Primernukleinsäuren und/oder Primerkomplementärnukleinsäuren bereitgestellt werden, ohne dass die Primernukleinsäure und/oder die Primerkomplementärnukleinsäure, insbesondere irreversibel, an die Lokalheizelemente funktionalisiert sein müssen. Somit können beispielsweise Lokalheizelemente, welche mit universellen Verbindungsnukleinsäuren funktionalisiert sind, in Kombination mit einer Vielzahl von verschiedenen Primernukleinsäuren und/oder Primerkomplementärnukleinsäuren verwendet werden, sofern die Verbindungsnukleinsäuren und die Primernukleinsäuren und/oder die Primerkomplementärnukleinsäuren aneinander binden können.

Dies bietet ferner den Vorteil, dass für neue Anwendungen, d.h. zum Vervielfältigen einer anderen Nukleinsäure, auf gegebenenfalls bereits vorhandene Lokalheizelemente, welche beispielsweise mit einer universellen Verbindungsnukleinsäure funktionalisiert sind, zurückgegriffen werden kann, und lediglich die Primernukleinsäure und/oder die Primerkomplementärnukleinsäure an die neue Anwendung bzw. an die zu vervielfältigende Nukleinsäure angepasst werden muss.

Gemäß einer bevorzugten Ausführungsform ist das zumindest eine Lokalheizelement als ein Nanopartikel ausgebildet und insbesondere dazu ausgelegt, durch eine Anregung Wärme an seine Umgebung zu übertragen.

Nanopartikel sind bevorzugt Partikel, die aufgrund ihrer Größe besondere optische Eigenschaften aufweisen, insbesondere charakteristische Absorptions- und/oder Streuspektren, die so im Volumenmaterial nicht oder nicht so deutlich hervortreten. Die Nanopartikel haben vorzugsweise einen Durchmesser zwischen 2 und 500 nm, besonders vorzugsweise zwischen 3 und 300 nm und ganz besonders vorzugsweise zwischen 5 und 200 nm. Bevorzugte Nanopartikel haben einen Durchmesser zwischen 7 und 150 nm. Die Nanopartikel können sphärisch sein, es kommen aber insbesondere auch nicht-globuläre Formen in Frage, z. B. elongierte Nanopartikel (Nanorods). In einer bevorzugten Ausführung umfasst der Nanopartikel mindestens einen Halbleiter oder ein Metall, vorzugsweise ein Edelmetall, z. B. Gold oder Silber. In einer Ausführung besteht der Nanopartikel vollständig aus dem Metall, in einer anderen bildet das Metall nur einen Teil des Nanopartikels, z. B. seine Hülle. Ein bevorzugter Nanopartikel kann ein Schale-Kern-Nanopartikel sein. Ein bevorzugter Nanopartikel kann an seiner Oberfläche Poren besitzen, die von Atomen oder Molekülen mit einer durch die Eigenschaften der Poren bestimmten Größe und Ladung besetzt werden können, besonders vorzugsweise lagern sich diese Atome oder Moleküle erst dann an den Nanopartikel an, wenn dieser sich in einer Lösung befindet. Erfindungsgemäß umfasst der Nanopartikel auch die an seiner Oberfläche angelagerten Atome und Moleküle. Bevorzugte Nanopartikel eignen sich aufgrund ihrer Materialabsorption oder Plasmonenresonanz dazu, optische Energie zu absorbieren.

Wenn durch Anregung eines Nanopartikels Wärme an seine Umgebung übertragen wird, bedeutet das, dass Energie auf den Nanopartikel und von dem Nanopartikel auf zumindest einen Teil der Reaktionslösung in der Umgebung des Nanopartikels übertragen wird, wobei der Nanopartikel durch die Übertragung der Energie seine Umgebung erhitzt. Dabei wird vorzugsweise durch die Anregung der Nanopartikel die unmittelbare Umgebung der Nanopartikel stärker erhitzt als die weitere Umgebung der Nanopartikel. Gewöhnlich werden die Nanopartikel zunächst durch Anregung erhitzt und übertragen dann Wärme an ihre Umgebung. Es ist jedoch auch denkbar, dass durch die Anregung der Nanopartikel Wärme an deren Umgebung übertragen wird, ohne dass die Nanopartikel zuerst selbst erhitzt werden.

Bevorzugt ist die Umgebung der Nanopartikel ein sphärisches Volumen, das den 100fachen Durchmesser des Nanopartikels hat, der sich in seinem Mittelpunkt befindet, besonders vorzugsweise hat es den 10fachen Durchmesser, ganz besonders vorzugsweise den 4fachen Durchmesser und vorzugsweise weniger als den 2fachen Durchmesser.

Insbesondere erfolgt eine lokale Erhitzung des Reaktionsvolumens in einer Umgebung, d.h. einem eingeschränkten Volumenbereich, um das Lokalheizelement bzw. um den Nanopartikel herum. Erfindungsgemäß kann ein einzelnes Lokalheizelement bzw. ein einzelnes Nanopartikel oder mehrere Lokalheizelemente bzw. mehrere Nanopartikel bereitgestellt werden. Die Anregung des Nanopartikels kann insbesondere mittels einer optischen Anregung erfolgen, d.h. mittels einer Zuführung von optischer Strahlung, welche zumindest teilweise von dem Nanopartikel absorbiert wird. Besonders bevorzugt kann die optische Anregung mittels Laserstrahlung erfolgen, wobei das Strahlungsspektrum der Laserstrahlung vorzugsweise derart gewählt wird, dass dieses zumindest teilweise mit einem Absorptionsspektrum des Nanopartikels überlappt. Die Verwendung von Laserstrahlung zur optischen Anregung des Nanopartikels kann insbesondere auch deshalb vorteilhaft sein, da insbesondere mittels gepulster Laserstrahlung eine Anregung des Nanopartikels für eine lediglich sehr kurze Zeitdauer und mit einer großen Wiederholrate erzielt werden kann, was für eine lokale Erhitzung zum Amplifizieren der Nukleinsäure sehr vorteilhaft sein kann. Eine detaillierte Beschreibung des lokalen Erhitzens zumindest eines Teils des Reaktionsvolumens unter Verwendung von Nanopartikeln und einer optischen Anregung mittels Laserstrahlung kann der bereits oben zitierten Druckschrift DE 10 2012 201 475 A1 entnommen werden.

Beispielsweise kann es zum Vervielfältigen der Nukleinsäure ausreichend sein, das Reaktionsvolumen mittels der Lokalheizelemente lokal zu erhitzen, wenngleich eine Kombination mit einer globalen Erhitzung des Reaktionsvolumens, beispielsweise durch herkömmliche Heizelemente, wie etwa Heizblöcke, möglich ist. Besonders bevorzugt können eine oder mehrere Verbindungsnukleinsäuren an eine Oberfläche des Nanopartikels gebunden sein. Dabei können bevorzugt auch verschiedene Verbindungsnukleinsäuren an die Oberfläche des Nanopartikels gebunden sein, beispielsweise jeweils mittels eines Thiollinkers.

Die Verwendung von Nanopartikeln als Lokalheizelemente bietet somit den Vorteil, dass Lokalheizelemente mit besonders kleinen Volumen und somit mit besonders kleiner thermischer Trägheit bereitgestellt werden können. Vorzugsweise befinden sich die Nanopartikel in direktem Kontakt mit der Reaktionslösung. Auf diese Weise können somit besonders schnelle Temperaturänderungen erzielt werden und folglich die Zeitdauer von thermischen Zyklen und oder die Zeitabstände zwischen zwei aufeinanderfolgenden thermischen Zyklen minimiert werden.

Ferner hat die Verwendung von Nanopartikeln den Vorteil, dass die Nanopartikel auf besonders einfache Weise hinsichtlich ihres Absorptionsspektrums an eine zu verwendende Anregungs-Lichtquelle angepasst werden können, um den Energieübertrag zu optimieren. Darüber hinaus bieten Nanopartikel, insbesondere Nanopartikel aus Gold und/oder anderen Edelmetallen oftmals den Vorteil, dass Nukleinsäuren auf einfache Weise an deren Oberfläche befestigt werden können, wie etwa mittels Thiollinkern.

Vorzugsweise ist das zumindest eine Lokalheizelement als ein Mikroheizelement ausgebildet und besonders bevorzugt dazu ausgelegt, durch ein resistives Heizen Wärme an seine Umgebung zu übertragen. Das Mikroheizelement befindet sich vorzugsweise in direktem Kontakt mit der Reaktionslösung bzw. dem Reaktionsvolumen. Beispielsweise kann ein Mikroheizelement in Form eines elektrisch leitfähigen Drahtes vorliegen. Besonders bevorzugt werden dabei Drähte verwendet, welche einen besonders dünnen Durchmesser aufweisen, wie etwa im Bereich von wenigen Mikrometern, um das Volumen und die thermische Trägheit des Mikroheizelements so gering wie möglich zu halten. Das Erhitzen der Umgebung der Mikroheizelemente kann vorzugsweise durch ein resistives Heizen der Mikroheizelemente erfolgen, was beispielsweise durch ein kontinuierliches und oder gepulstes Bestromen der Mikroheizelemente erzielt werden kann. Besonders bevorzugt sind die Mikroheizelemente aus einem Edelmetall, wie etwa Gold und/oder Silber, ausgebildet und/oder damit beschichtet, um ein einfaches Konjugieren bzw. Funktionalisierten einer Oberfläche des Mikroheizelements mit einer oder mehreren Verbindungsnukleinsäuren zu ermöglichen.

Die Verwendung eines Mikroheizelements als Lokalheizelement, insbesondere die Verwendung eines resistives geheizten Drahtes, bietet den Vorteil, dass ein lokales Heizen der Umgebung des jeweiligen Mikroheizelements mit besonders einfachen technischen Mitteln erreicht werden kann, da zum Heizen des Mikroheizelements im Wesentlichen lediglich ein kontrolliertes Bestromen des Mikroheizelements vorgenommen werden muss. Das Bestromen erfolgt vorzugsweise mittels einer externen Spannungsquelle und/oder Stromquelle. Gemäß manchen bevorzugten Ausführungsformen können eine oder mehrere Batterien als eine externe Spannungsquelle ausreichend sein, um das zumindest eine Mikroheizelement zu beheizen bzw. zu bestromen. Insbesondere kann das Bestromen derart erfolgen, dass lokal in einer Umgebung des Mikroheizelements die Denaturierungstemperatur erreicht und/oder überschritten wird, während in größerer Entfernung vom Mikroheizelement das Reaktionsvolumen bzw. die Reaktionslösung eine niedrigere Temperatur aufweist, welche vorzugsweise auf der für die Vervielfältigung der Nukleinsäure erforderlichen Elongations- und/oder Annealingtemperatur gehalten wird. Beispielsweise ist es möglich, eine Mehrzahl von Lokalheizelementen bereitzustellen, welche jeweils als Nanopartikel oder als resistives Mikroheizelement bzw. als Mikrodraht ausgebildet sind. Insbesondere können eine Mehrzahl von Nanopartikeln als Lokalheizelemente bereitgestellt werden und/oder eine Mehrzahl von Mikroheizelementen als Lokalheizelemente bereitgestellt werden. Dabei können beispielsweise die Nanopartikel mittels optischer Anregung geheizt werden und die Mikroheizelemente mittels Bestromung geheizt werden.

Bevorzugt kann ein Lokalheizelement, insbesondere ein Mikroheizelement, zusätzlich dazu ausgelegt sein, bei entsprechender Anregung und/oder Bestromung zumindest einen Teil der Reaktionslösung global zu erhitzen, d.h. nicht nur eine Umgebung um das Lokalheizelement herum zu erhitzen. Dies kann beispielsweise durch ein stärkeres und/oder längeres resistives Heizen bzw. Bestromen des Mikroheizelements erfolgen, so dass sich beispielsweise die Umgebung um das Mikroheizelement herum, welche dadurch beheizt wird vergrößert, bis gegebenenfalls die Umgebungen mehrerer benachbarter Mikroheizelemente einander überlappen. Ferner kann zum globalen Heizen das Bestromen bzw. das resistive Heizen des zumindest einen Mikroheizelements derart erfolgen, dass eine Änderung der mittleren Temperatur der Reaktionslösung, insbesondere auch außerhalb der Umgebung des zumindest einen Mikroheizelements, erfolgt.

Besonders bevorzugt steht das zumindest eine Lokalheizelement in direktem Kontakt mit der Reaktionslösung bzw. dem Reaktionsvolumen. Insbesondere können mehrere Lokalheizelemente als kolloidale Nanopartikel in der Reaktionslösung bzw. im Reaktionsvolumen bereitgestellt werden und/oder ein oder mehrere Lokalheizelemente als Mikroheizelemente, insbesondere als Mikrodrähte, zumindest teilweise durch die Reaktionslösung bzw. durch das Reaktionsvolumen verlaufend bereitgestellt werden. Dies bietet den Vorteil, dass eine thermische Trägheit so gering wie möglich gehalten wird, da sich in direkter Umgebung der Lokalheizelemente, welche mittels lokalem Heizen geheizt werden, zumindest ein Teil der Reaktionslösung mit der zu vervielfältigen Nukleinsäure befindet.

Die Primernukleinsäure weist zumindest einen Primerabschnitt auf, welcher zum Binden an die Nukleinsäure ausgelegt ist, und weist zumindest einen Verbindungsabschnitt auf, welcher zum Binden an die zumindest eine Verbindungsnukleinsäure ausgelegt ist. Mit anderen Worten weist die Primernukleinsäure zumindest zwei Abschnitte auf, umfassend einen Primerabschnitt und einen Verbindungsabschnitt. Die Primernukleinsäure kann als ein Oligonukleotid ausgebildet sein, wobei vorzugsweise der Primarabschnitt und oder der Verbindungsabschnitt jeweils einen Teil des Oligonukleotids bilden. Der Verbindungsabschnitt ist dabei vorzugsweise derart ausgelegt, dass dieser ein Binden der Primernukleinsäure an eine Verbindungsnukleinsäure ermöglicht. Insbesondere kann der Verbindungsabschnitt dazu ausgelegt sein, mit der Verbindungsnukleinsäure zumindest teilweise zu hybridisieren. Der Primerabschnitt ist vorzugsweise dazu ausgelegt, als ein Primer zum Vervielfältigen der Nukleinsäure zu fungieren.

Insbesondere kann der Primerabschnitt dazu ausgelegt sein, als Vorwärtsprimer und/oder als Rückwärtsprimer zu agieren.

Unabhängig von der Primernukleinsäure, welche dazu ausgelegt ist, an die Verbindungsnukleinsäure zu binden, kann gemäß einer bevorzugten Ausführungsform zumindest ein weiterer Primer in der Reaktionslösung vorhanden sein, welcher nicht dazu ausgelegt ist an die Verbindungsnukleinsäure zu binden. Beispielsweise kann ein Vorwärtsprimer als Primernukleinsäure ausgestaltet sein und dazu ausgelegt sein, an die Verbindungsnukleinsäure zu binden, während ein Rückwärtsprimer nicht dazu ausgelegt ist, an die Verbindungsnukleinsäure zu binden, oder umgekehrt.

Vorzugsweise ist eine Nukleotidsequenz in dem Verbindungsabschnitt der Primernukleinsäure zumindest teilweise komplementär zu einer Nukleotidsequenz der Verbindungsnukleinsäure und/oder eine Nukleotidsequenz in dem Primerabschnitt zumindest teilweise komplementär zu einer Nukleotidsequenz der Nukleinsäure. Dies bietet den Vorteil, dass die Primernukleinsäure mit dem Verbindungsabschnitt mit der Verbindungsnukleinsäure hybridisieren kann und/oder dass die Primernukleinsäure mit dem Primerabschnitt mit der zu vervielfältigenden Nukleinsäure hybridisieren kann.

Vorzugsweise weist die Primerkomplementärnukleinsäure zumindest einen Verbindungsabschnitt auf, welcher dazu ausgelegt ist, an die zumindest eine Verbindungsnukleinsäure zu binden. Weiter bevorzugt weist die Primerkomplementärnukleinsäure zumindest einen Primerkomplementärabschnitt auf, welcher dazu ausgelegt ist, die Verbindungsnukleinsäure mittels einer enzymatischen Reaktion um eine Primernukleotidsequenz zu elongieren. Mit anderen Worten kann der Primerkomplementärabschnitt vorzugsweise als eine Vorlage für eine Erzeugung einer Primernukleotidsequenz dienen, wobei die Verbindungsnukleinsäure mittels einer enzymatischen Reaktion um eine Primernukleotidsequenz elongiert wird. Mit anderen Worten weist die Primerkomplementärnukleinsäure vorzugsweise einen Verbindungsabschnitt auf, mittels welchem die Primerkomplementärnukleinsäure an die Verbindungsnukleinsäure binden kann, und ferner einen Primerkomplementärabschnitt, welcher als Vorlage für eine Erzeugung einer Primernukleotidsequenz dienen kann. Die Primernukleotidsequenz kann dabei als Primer für eine Vervielfältigungsreaktion, insbesondere für eine PCR, zur Vervielfältigung der Nukleinsäure dienen und ist besonders bevorzugt zumindest teilweise komplementär zur Nukleinsäure. Besonders bevorzugt ist die Primerkomplementärnukleinsäure derart ausgelegt, dass wenn die Primerkomplementärnukleinsäure mittels des Verbindungsabschnitts an die Verbindungsnukleinsäure gebunden ist, die Verbindungsnukleinsäure mittels einer enzymatischen Reaktion, wie etwa mittels einer Polymerase, um einen Primerabschnitt bzw. um eine Primer-Nukleotidsequenz erweitert bzw. elongiert werden kann, sodass die elongierte Verbindungsnukleinsäure zumindest teilweise als Primer zum Vervielfältigen der Nukleinsäure dienen kann. Mit anderen Worten kann die Primerkomplementärnukleinsäure vorzugsweise dazu dienen, die mit Verbindungsnukleinsäuren funktionalisierten Lokalheizelemente mittels einer enzymatischen Reaktion derart zu individualisieren, dass diese als Primer bzw. Primernukleinsäuren zum Vervielfältigen der gewünschten Nukleinsäure dienen können. Dies hat den Vorteil, dass Lokalheizelemente, welche mit einer universellen Verbindungsnukleinsäure funktionalisiert sind, verwendet werden können, welche erst in der Reaktionslösung bzw. im Reaktionsvolumen und/oder zeitlich vor, insbesondere unmittelbar vor, der eigentlichen Amplifikationsreaktion mittels einem Bereitstellen einer oder mehrerer geeigneter Primerkomplementärnukleinsäuren individualisiert werden können, d.h. an die spezifische, zu vervielfältigende Nukleinsäure angepasst werden können. Ferner ermöglicht dies, Lokalheizelemente, welche mit, insbesondere universellen, Verbindungsnukleinsäuren funktionalisiert sind, mittels unterschiedlicher Primerkomplementärnukleinsäuren zu individualisieren, sodass diese beispielsweise zum, insbesondere gleichzeitigen, Vervielfältigen unterschiedlicher Nukleinsäuren angepasst sind.

Vorzugsweise ist eine Nukleotidsequenz in dem Verbindungsabschnitt der Primerkomplementärnukleinsäure zumindest teilweise komplementär zu einer Nukleotidsequenz der Verbindungsnukleinsäure und/oder eine Nukleotidsequenz in dem Primerkomplementärabschnitt zumindest teilweise komplementär zu der Primernukleotidsequenz. Dies ermöglicht ein Hybridisieren der Primerkomplementärnukleinsäure an die Verbindungsnukleinsäure und/oder ein Hybridisieren der durch enzymatische Elongation der Verbindungsnukleinsäure entstandenen Primernukleotidsequenz an die zu vervielfältigende Nukleinsäure.

Gemäß einer bevorzugten Ausführungsform werden mehrere Lokalheizelemente bereitgestellt, welche jeweils mit mehreren Verbindungsnukleinsäuren funktionalisiert sind. Dabei kann jedes Lokalheizelement mit mehreren gleichartigen oder unterschiedlichen Verbindungsnukleinsäuren funktionalisiert sein, wobei sich die Verbindungsnukleinsäuren beispielsweise in ihrer Nukleotidsequenz unterscheiden. Ferner können erste Lokalheizelemente bereitgestellt werden, welche mit Verbindungsnukleinsäuren einer ersten Art funktionalisiert sind, und zweite Lokalheizelemente, welche mit Verbindungsnukleinsäuren einer zweiten Art funktionalisiert sind. Dies kann beispielsweise ermöglichen, in einer Reaktionslösung bzw. in einem Reaktionsvolumen mehrere unterschiedliche Nukleinsäuren im Wesentlichen gleichzeitig zu vervielfältigen. Mit anderen Worten kann dies dazu dienen, ein Multiplexing bei der Vervielfältigung von Nukleinsäuren zu erzielen.

Vorzugsweise weist die Primernukleinsäure zwischen dem zumindest einen Verbindungsabschnitt und dem zumindest einen Primerabschnitt zumindest eine abasische Modifikation auf. Dies bietet den Vorteil, dass eine Trennung bzw. eine Zäsur zwischen dem Verbindungsabschnitt und dem Primerabschnitt vorliegt. Insbesondere kann die zumindest eine abasische Modifikation bewirken, dass sich eine enzymatische Reaktion, wie beispielsweise das Elongieren des Primerabschnitts entlang der zu vervielfältigenden Nukleinsäure mittels einer Polymerase, vorzugsweise nicht auf den Verbindungsabschnitt erstreckt. Mit anderen Worten kann das Bereitstellen zumindest einer abasischen Modifikation, bevorzugt mehrerer abasischer Modifikationen, den Verlauf einer enzymatischen Reaktion unterbrechen und/oder beenden, um spezifische Bereiche, insbesondere der Verbindungsabschnitte, von der enzymatischen Reaktion auszuschließen.

Insbesondere in einer Ausführungsform, in welcher die an Lokalheizelemente funktionalisierten Verbindungsnukleinsäuren entsprechend der Primerkomplementärnukleinsäuren um einen Primerabschnitt bzw. eine Primernukleotidsequenz elongiert wurden, und die an die Verbindungsnukleinsäuren gebundenen Primerkomplementärnukleinsäuren nach einem Erhitzen der jeweiligen Lokalheizelemente wieder gelöst bzw. entfernt wurden, kann es hilfreich sein, dass die Verbindungsnukleinsäuren eine oder mehrere abasische Modifikationen aufweisen. Hierdurch kann eine oder mehrere abasische Modifikationen zwischen einem Teil der ursprünglichen Verbindungsnukleinsäure und dem hinzugekommenen Primerabschnitt bereitgestellt werden, wodurch vorzugsweise ein Teil der ursprünglichen Verbindungsnukleinsäure von der enzymatischen Reaktion ausgeschlossen werden kann. Vorzugsweise weist die Verbindungsnukleinsäuren die mindestens eine abasische Modifikation zwei Nukleotidbasen vom 3'-Ende entfernt, weiter bevorzugt drei Nukleotidbasen vom 3'-Ende entfernt, besonders bevorzugt vier Nukleotidbasen vom 3'-Ende entfernt und ganz besonders bevorzugt mindestens fünf Nukleotidbasen vom 3'-Ende entfernt auf. Dies kann vorteilhaft sein, um der Polymerase für die Elongation am 3'-Ende der Verbindungsnukleinsäuren einen kurzen Teil doppelsträngiger Nukleinsäure als Start- und/oder Bindestelle für eine Polymerase bereitzustellen.

Gemäß einer weiteren bevorzugten Ausführungsform kann die Verbindungsnukleinsäure zumindest eine abasische Modifikation aufweisen, welche vorzugsweise zumindest wenige Nukleotidbasen von dem 3'-Ende der Verbindungsnukleinsäure entfernt angeordnet ist, beispielsweise weniger als 20 Nukleotidbasen, bevorzugt weniger als zehn Nukleotidbasen. Dies hat den Vorteil, dass die vorzugsweise wenigen Nukleotidbasen zwischen dem 3'-Ende der Verbindungsnukleinsäure und der abasischen Modifikation mit einer an die Verbindungsnukleinsäure gebundenen Primerkomplementärnukleinsäure über zumindest wenige Nukleotidsequenzen einen Doppelstrang bilden und somit beispielsweise eine Start- und/oder Bindestelle für eine Polymerase bereitstellen. Dies bietet den Vorteil, dass zwar der Primerkomplementärabschnitt beispielsweise von einer Polymerase komplettiert werden kann, während ein Wirken der Polymerase an der abasischen Modifikation unterbrochen wird.

Gemäß einer weiteren bevorzugten Ausführungsform kann eine Mehrzahl von, insbesondere unterschiedlichen, Primernukleinsäuren bereitgestellt werden, deren Primerabschnitte als Vorwärtsprimer und/oder als Rückwärtsprimer ausgebildet sind. Insbesondere können die Lokalheizelemente und/oder die Verbindungsnukleinsäuren und/oder die Primernukleinsäuren und/oder die Primerkomplementärnukleinsäuren derart ausgebildet sein, dass an manchen Lokalheizelementen ausschließlich Vorwärtsprimer ausgebildet sind und/oder an anderen Lokalheizelementen lediglich Rückwärtsprimer ausgebildet sind. Dadurch kann beispielsweise erreicht werden, dass eine zu vervielfältigende Nukleinsäure an einem Ende an ein Lokalheizelement mit Vorwärtsprimer gebunden ist und an dem anderen Ende an ein Lokalheizelement mit Rückwärtsprimer gebunden ist. Dadurch kann vorzugsweise mittels der beiden Lokalheizelemente das Beheizen der Nukleinsäure, insbesondere auf und/oder über die Denaturierungstemperatur, besonders effizient erfolgen, da von zwei Seiten Wärme auf die Nukleinsäure einwirken kann.

Alternativ oder zusätzlich können an manchen Lokalheizelementen sowohl Vorwärtsprimer als auch Rückwärtsprimer ausgebildet ein. Dies bietet den Vorteil, dass eine zu vervielfältigende Nukleinsäure mit beiden Enden an das gleiche Lokalheizelement gebunden werden kann. Dies ermöglicht ein besonders effektives Beheizen der Nukleinsäure, insbesondere über die Denaturierungstemperatur, da der mittlere Abstand der Nukleinsäure von dem Lokalheizelement verringert bzw. begrenzt werden kann.

Gemäß der Erfindung ist das Lokalheizelement dazu vorgesehen, seine Umgebung zu erhitzen und insbesondere ein begrenztes Teilvolumen des Reaktionsvolumens in seiner Umgebung vorzugsweise auf eine Temperatur größer oder gleich der Denaturierungstemperatur zu erhitzen. Sofern die Denaturierungstemperatur erreicht oder überschritten wird, wird vorzugsweise eine über die Verbindungsnukleinsäure und gegebenenfalls über die Primernukleinsäure mit dem Lokalheizelement verbundene in einer enzymatischen Reaktion elongierte Nukleinsäure gelöst, sodass diese nicht mehr über die Verbindungsnukleinsäure und gegebenenfalls über die Primernukleinsäure mit dem Lokalheizelement verbunden ist. Ferner werden doppelsträngig vorliegende Nukleinsäuren zumindest teilweise in Einzelstränge aufgetrennt. Sofern die Primernukleinsäure mittels Hybridisierung an die Verbindungsnukleinsäure gebunden ist, ist nicht auszuschließen, dass sich auch die Primernukleinsäure bei Erreichen oder Überschreiten der Denaturierungstemperatur von der Verbindungsnukleinsäure löst. Dies steht im Allgemeinen dem Funktionieren des erfindungsgemäßen Systems und/oder Verfahrens nicht im Wege, da nach dem Lösen der Primernukleinsäure von der Verbindungsnukleinsäure entweder dieselbe Primernukleinsäure bei niedrigeren Temperaturen als der Denaturierungstemperatur wieder an die Verbindungsnukleinsäure binden kann oder eine andere Primernukleinsäure an die freigewordene Verbindungsnukleinsäure binden kann. Sofern gewünscht ist, dass dieselbe Primernukleinsäure wieder mit der Verbindungsnukleinsäure bindet, an welcher sie zuvor gebunden war, kann es vorteilhaft sein, eine Zeitdauer des Anregens bzw. Beheizens des Lokalheizelements möglichst kurz zu halten, sodass zwar eine Denaturierung stattfinden kann, aber die Temperatur in der Umgebung des Lokalheizelements möglichst schnell wieder unter die Denaturierungstemperatur fällt, um die Zeitdauer, welche die Primernukleinsäure zum Wegdiffundieren bzw. zum sich Entfernen von der Verbindungsnukleinsäure zur Verfügung hat, zu minimieren. Da oftmals für eine Denaturierung eine Zeitdauer von wenigen Mikrosekunden ausreichend ist, kann es somit vorteilhaft sein, auch das Beheizen bzw. Anregen des Lokalheizelements über eine Zeitdauer von nur wenigen Mikrosekunden vorzunehmen. Vorzugsweise erfolgt das Beheizen bzw. Anregen des zumindest einen Lokalheizelements für einen Denaturierungsschritt für weniger als 1 ms, weiter bevorzugt für weniger als 500 µs, mehr bevorzugt für weniger als 250 µs, noch mehr bevorzugt für weniger als 100 µs, viel mehr bevorzugt für weniger als 50 µs, sehr viel mehr bevorzugt für weniger als 25 µs, am meisten bevorzugt für weniger als 10 µs.

Jedoch kann es für andere bevorzugte Ausführungsformen wünschenswert sein, ein Ablösen der Primernukleinsäure von der Verbindungsnukleinsäure möglichst zu vermeiden bzw. zu unterbinden. Dazu können gemäß bevorzugter Ausführungsformen die Verbindungsnukleinsäure und/oder die Primernukleinsäure, vorzugsweise jeweils, zumindest ein Immobilisierungselement aufweisen, welches dazu ausgelegt ist, die Primernukleinsäure, wenn an die Verbindungsnukleinsäure gebunden, derart an der Verbindungsnukleinsäure zu immobilisieren, dass die Primernukleinsäure auch während und/oder nach einem Denaturierungsschritt an die Verbindungsnukleinsäure gebunden bleibt. Das zumindest eine Immobilisierungselement kann beispielsweise als zumindest eine Modifikation in der Nukleotidsequenz der Verbindungsnukleinsäure und/oder in der Nukleotidsequenz der Primernukleinsäure, insbesondere im Verbindungsabschnitt der Primernukleinsäure, ausgebildet sein, wobei die zumindest eine Modifikation beispielsweise zumindest eine Amino-Gruppe aufweist. Die zumindest eine Modifikation kann vorzugsweise sodann dazu führen, dass mittels einer chemischen Reaktion, etwa mittels eines d-Linkers, die Verbindungsnukleinsäure und die Primernukleinsäure derart ausreichend fest mit einander verbunden werden, dass sich diese auch bei einer Temperatur größer und/oder gleich der Denaturierungstemperatur nicht voneinander lösen. Sofern ein derartiges Immobilisieren der Primernukleinsäure an die Verbindungsnukleinsäure gewünscht ist, kann es vorteilhaft sein, das Immobilisieren vor einer Durchführung des Verfahrens zur Vervielfältigung der Nukleinsäure durchzuführen, insbesondere bevor erstmals mittels des zumindest einen Lokalheizelements die Umgebung des Lokalheizelements auf die Denaturierungstemperatur erhitzt wird. Ferner kann es vorteilhaft sein, etwaige überschüssige Primernukleinsäure, welche nicht an eine Verbindungsnukleinsäure gebunden sind, nach dem Immobilisieren und vorzugsweise vor der Durchführung des Verfahrens zum Vervielfältigen aus der Reaktionslösung zu entfernen. Beispielsweise kann das Entfernen der ungebundenen Primernukleinsäuren mittels einem Zentrifugieren und/oder einer Filtration erfolgen.

Gemäß einer weiteren Ausführungsform wird zeitlich vor dem Durchführen einer Vervielfältigungsreaktion, wie etwa einer PCR, noch zumindest eine weitere Reaktion durchgeführt. Die zumindest eine weitere Reaktion kann beispielsweise eine Reverse Transkription (RT) umfassen, bei der z.B. durch eine enzymatische Reaktion ein Typ von Nukleinsäure wie z.B. RNA in einen anderen Typ von Nukleinsäure wie z.B. DNA umgeschrieben wird.

Primerkomplementärnukleinsäuren und/oder Primernukleinsäuren können vorzugsweise jeweils in einer Konzentration von mehr als 1nM, besonders vorzugsweise mehr als 5nM und ganz besonders vorzugsweise mehr als 25nM vorliegen, und/oder vorzugsweise in einer Konzentration von weniger als 1000nM, besonders bevorzugt weniger als 300nM und ganz besonders bevorzugt weniger als 100nM.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand von Ausführungsbeispielen in den Zeichnungen schematisch dargestellt und wird im Folgenden unter Bezugnahme auf die Zeichnungen beschrieben.

### Figurenbeschreibung

Die Figuren 1A bis 1E zeigen in schematischen Darstellungen eine erste bevorzugte Ausführungsform der Erfindung.

Figur 2 zeigt in einer schematischen Darstellung einen beispielhaften Verfahrensablauf gemäß der ersten bevorzugten Ausführungsform.

Die Figuren 3A bis 3C zeigen in schematischen Darstellungen Erläuterungen zu Teilaspekten der ersten bevorzugten Ausführungsform.

Die Figuren 4A bis 4D zeigen in schematischen Darstellungen eine zweite bevorzugte Ausführungsform der Erfindung.

Figur 5 zeigt in einer schematischen Darstellung einen beispielhaften Verfahrensablauf gemäß der zweiten bevorzugten Ausführungsform.

Figur 6 zeigt experimentelle Daten zum ersten Beispiel.

Figur 7 zeigt experimentelle Daten zum zweiten Beispiel.

Figur 8 zeigt experimentelle Daten zum dritten Beispiel.

Figur 9 zeigt experimentelle Daten zum vierten Beispiel.

### Detaillierte Beschreibung der Zeichnungen

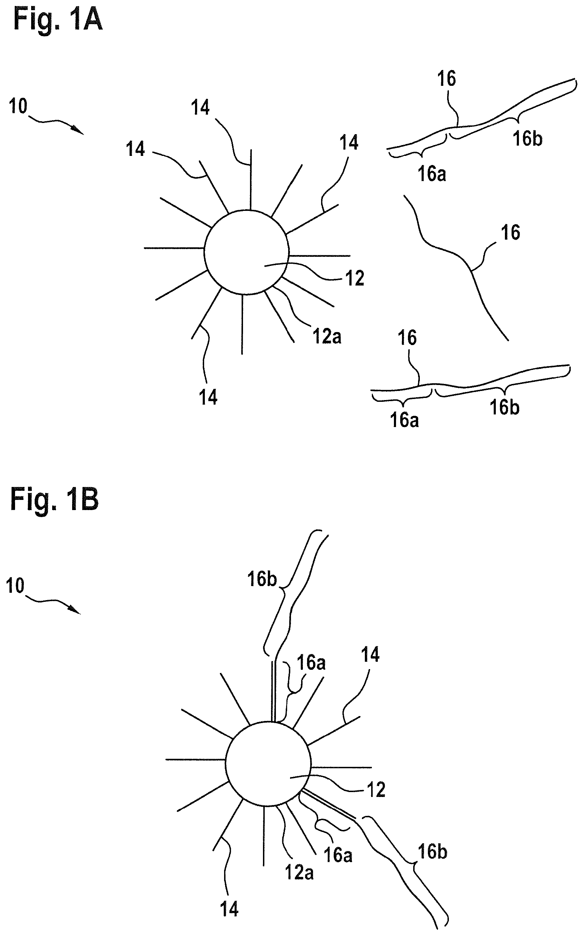

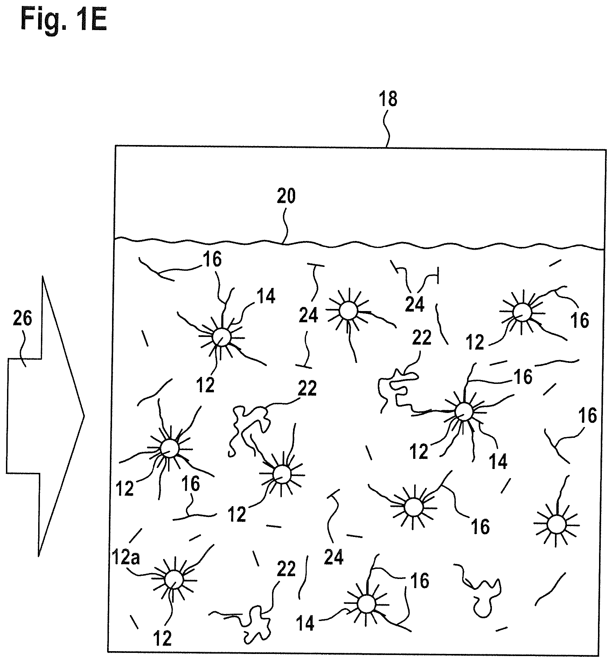

Die Figuren 1A bis 1E zeigen in einer schematischen Darstellung eine erste bevorzugte Ausführungsform eines Systems 10 zum Vervielfältigen einer Nukleinsäure, wobei das System zumindest ein Lokalheizelement 12 aufweist, welches mit mehreren Verbindungsnukleinsäuren 14 funktionalisiert ist. Besonders bevorzugt sind die Verbindungsnukleinsäuren 14 ausreichend fest an dem Lokalheizelement 12 befestigt, so dass diese auch an dem Lokalheizelement 12 befestigt bleiben, wenn eine Temperatur des Lokalheizelements 12 und/oder eine Temperatur der Umgebung des Lokalheizelements 12 eine Denaturierungstemperatur erreicht und/oder übersteigt. Die Verbindungsnukleinsäuren 14 können dabei gleichartig und/oder unterschiedlich ausgebildet sein. Des Weiteren weist das System 10 eine Mehrzahl von Primernukleinsäuren 16 auf, welche separat von dem Lokalheizelement 12 und den Verbindungsnukleinsäuren 14 bereitgestellt werden. Die Primernukleinsäuren 16 weisen jeweils einen Verbindungsabschnitt 16a und einen Primerabschnitt 16b auf. Insbesondere ist das System 10 dazu ausgelegt, in einem Reaktionsvolumen, insbesondere in einer Reaktionslösung 20 (siehe Figur 1E), bereitgestellt zu werden. Besonders bevorzugt werden eine Vielzahl von Systemen 10 in der Reaktionslösung bereitgestellt.

Die Figuren 1A und 1B zeigen dabei ein System 10, bei welchem das Lokalheizelement 12 als ein Nanopartikel 12a ausgebildet ist. Insbesondere kann dabei in der Reaktionslösung 20 eine Mehrzahl von Systemen 10 bereitgestellt werden, wobei die Nanopartikel 12a vorzugsweise als kolloidale Nanopartikel 12a in der Reaktionslösung vorliegen. Die Verbindungsnukleinsäuren 14 sind an der Oberfläche des Nanopartikels 12a befestigt, beispielsweise mittels Thiollinkern. Wenn die funktionalisierten Nanopartikel 12a und die Primernukleinsäuren 16 separat in der Reaktionslösung bereitgestellt werden, liegen diese zumindest teilweise getrennt voneinander in der Reaktionslösung vor, wie beispielsweise in Figur 1A dargestellt. Sofern die Temperatur der Reaktionslösung, insbesondere in der Umgebung um das zumindest eine Lokalheizelement 12 bzw. um das zumindest eine Nanopartikel 12a niedriger als die Denaturierungstemperatur ist, vorzugsweise deutlich niedriger als die Denaturierungstemperatur, und besonders bevorzugt in etwa der Annealingtemperatur entspricht, können Primernukleinsäuren 16 der Mehrzahl von Primernukleinsäuren 16 mit den Verbindungsnukleinsäuren 14 binden, sodass zumindest ein Teil der Primernukleinsäuren 16 über die Verbindungsnukleinsäuren 14 mit dem Nanopartikel 12a verbunden ist. Die Zeit, welche erforderlich ist, bis ein bestimmter Teil bzw. eine bestimmte Anzahl von Primernukleinsäuren 16 an die funktionalisierten Nanopartikel 12a bindet, kann dabei von verschiedenen Parametern abhängen, wie etwa einer Temperatur der Reaktionslösung, sowie einer Konzentration der Primernukleinsäuren 16 und/oder der funktionalisierten Nanopartikeln 12a in der Reaktionslösung, und/oder einer Besatzdichte, mit welcher die Verbindungsnukleinsäuren 14 auf den Nanopartikeln 12a befestigt sind und/oder einer Konzentration von Salzen.

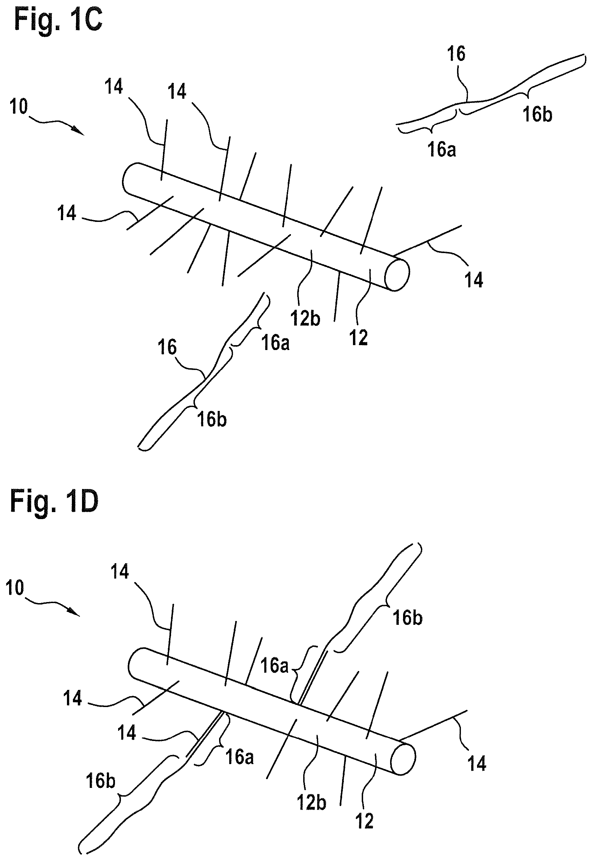

Die Figuren 1C und 1D zeigen eine Abwandlung der ersten bevorzugten Ausführungsform des Systems 10, welche in den wesentlichen Teilen der in den Figuren 1A und 1B gezeigten Ausführungsform entspricht, wobei abweichend davon das Lokalheizelement 12 als ein resistives Mikroheizelement 12b ausgebildet ist. Insbesondere kann das Mikroheizelement 12b als ein Mikrodraht ausgebildet sein. Wenngleich die Figuren 1C und 1D lediglich einen kurzen Abschnitt eines Mikroheizelements 12b schematisch darstellen, kann eine Länge des Mikroheizelements 12b deutlich länger sein, als dargestellt. Das Mikroheizelement 12b kann beispielsweise dadurch erhitzt werden, dass das Mikroheizelement 12b derart bestromt wird, dass ein Stromfluss im Wesentlichen entlang einer Drahtlängsachse durch das Mikroheizelement 12b fließt und dieses erhitzt. Entsprechend der vorherigen bevorzugten Ausführungsform sind mehrere Verbindungsnukleinsäuren 14 an der Oberfläche des Mikroheizelements 12b befestigt, beispielsweise mittels Thiollinkern.

Figur 1E zeigt in einer schematischen Darstellung ein Reaktionsgefäß 18, welches eine Reaktionslösung 20 beinhaltet. In der Reaktionslösung 20 ist eine Mehrzahl von Lokalheizelementen 12, welche jeweils als Nanopartikel 12a ausgebildet sind, bereitgestellt, und welche jeweils mit mehreren Verbindungsnukleinsäuren 14 funktionalisiert sind. Ferner ist in der Reaktionslösung 20 eine Mehrzahl von Primernukleinsäuren 16 bereitgestellt, welche sich zum Teil frei in der Reaktionslösung 20 befinden und zum Teil über Verbindungsnukleinsäuren 14 an die Nanopartikel 12a gebunden sind. Wie ferner dargestellt ist, können die Primernukleinsäuren 16 über die jeweiligen Primerabschnitte 16b mit einer der zu vervielfältigenden, einzelsträngig vorliegenden Nukleinsäuren 22 hybridisieren und daran binden. Dies gilt im Wesentlichen in gleichem Maße für Primernukleinsäuren 16, welche an Verbindungsnukleinsäuren 14 gebunden sind, und für Primernukleinsäuren 16, welche nicht an Verbindungsnukleinsäuren 14 gebunden sind, sondern sich frei in der Reaktionslösung 20 befinden. Darüber hinaus können auch noch weitere Primer 24 und oder Primernukleinsäuren in der Reaktionslösung 20 vorhanden sein. Beispielsweise können diese weiteren Primer 24 als Rückwärtsprimer ausgebildet sein während die Primernukleinsäuren 16 bzw. deren Primerabschnitte 16b als Vorwärtsprimer ausgebildet sind, oder umgekehrt. Auch ist es möglich, dass weitere Primer 24 in der Reaktionslösung 20 bereitgestellt werden, welche nicht über einen Verbindungsabschnitt 16a verfügen und demnach nicht an die Verbindungsnukleinsäure 14 binden können. Beispielsweise können diese weiteren Primer 24 mit den an die Nanopartikel 12a bindbaren Primernukleinsäuren 16 konkurrieren.

Damit die zu vervielfältigen Nukleinsäure 22 in der Reaktionslösung 20 als einzelsträngigen Nukleinsäure 22 vorliegt, kann es vorteilhaft sein, zu Beginn oder vor einer durchzuführenden Vervielfältigung der Nukleinsäure 22 eine globale Erwärmung bzw. Erhitzung der Reaktionslösung 20 auf und/oder über die Denaturierungstemperatur vorzunehmen, um die ggf. doppelsträngig vorliegenden Nukleinsäuren 22 zunächst in Einzelstränge aufzutrennen. Ferner kann ein Erhitzen der Reaktionslösung zu Beginn und/oder vor einer durchzuführenden Vervielfältigung der Nukleinsäure 22, beispielsweise durch eine globale Erwärmung bzw. Erhitzung der Reaktionslösung 20, vorteilhaft oder gar notwendig sein, insbesondere wenn beispielsweise sogenannte HotStart-Enzyme verwendet werden, welche erst durch eine Hitzeeinwirkung aktiviert werden müssen und/oder wenn Komponenten, wie etwa Enzyme, vor der eigentlichen Vervielfältigung der Nukleinsäure 22 deaktiviert werden sollen bzw. müssen.

Die zur PCR bzw. zur Vervielfältigung der Nukleinsäuren 22 notwendige Denaturierung erfolgt dabei durch eine optische Anregung der Nanopartikel 12a, wodurch die Nanopartikel 12a und/oder eine lokale Umgebung der Nanopartikel 12a auf und/oder über die Denaturierungstemperatur erhitzt wird, während im Wesentlichen der Rest der Reaktionslösung 22 nicht durch die optische Anregung 26 der Nanopartikel 12a erhitzt wird, sondern auf einer niedrigeren Temperatur verbleibt. Besonders bevorzugt ist diese niedrigere Temperatur der Reaktionslösung derart gewählt, dass diese im Wesentlichen einer Annealingtemperatur entspricht, welche ein Elongieren mittels der Polymerase und/oder ein Hybridisieren von einzelsträngigen Nukleinsäuren und/oder einer Bindung von Primernukleinsäuren 16 an Verbindungsnukleinsäuren 14 ermöglicht. Die optische Anregung 26 kann beispielsweise durch ein Einstrahlen von optischer Strahlung in die Reaktionslösung 20 erfolgen, wozu vorteilhafter Weise das Reaktionsgefäß 18 zumindest teilweise transparent für die optische Strahlung ist, wobei die optische Strahlung spektral zumindest teilweise mit einem Absorptionsspektrum der Nanopartikel 12a überlappt und derart gewählt ist, dass diese im Wesentlichen nicht von der Reaktionslösung, abgesehen von den Nanopartikeln 12a in der Reaktionslösung, absorbiert wird. Dabei versteht sich, dass die in Figur 1E gezeigte Ausführung auch mit Mikroheizelementen 12b als Lokalheizelemente 12 in Kombination mit oder anstatt von Nanopartikeln 12a verwendet werden kann, wenngleich dies nicht in einer eigenen Figur dargestellt ist.

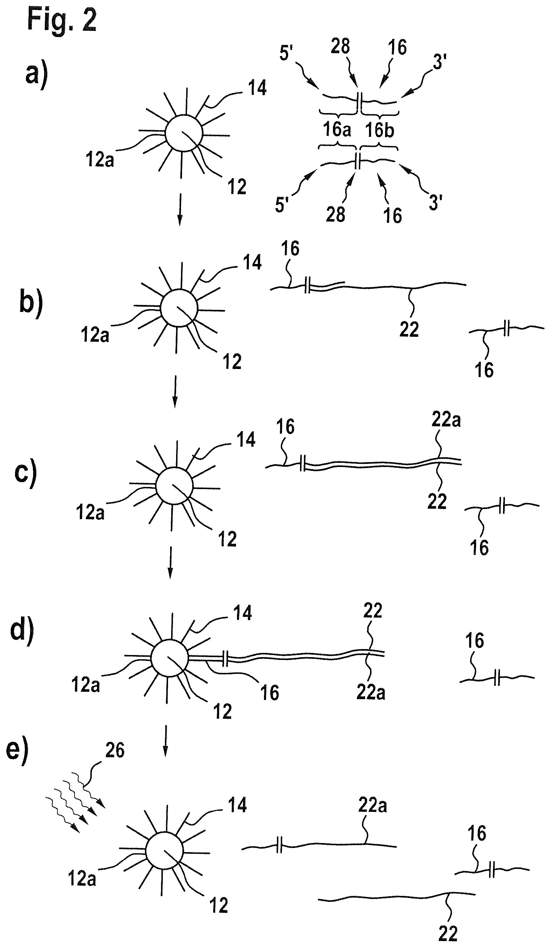

Im Folgenden wird die erste bevorzugte Ausführungsform mit Bezug auf Figur 2 im Detail erläutert, insbesondere ein Verfahren zum Vervielfältigen der Nukleinsäure gemäß der ersten bevorzugten Ausführungsform.

Gemäß der ersten bevorzugten Ausführungsform werden in der Reaktionslösung 20 zu Beginn in Schritt a) zumindest eine Lokalheizelement 12 und zumindest eine Primernukleinsäure 16 separat voneinander bereitgestellt. Vorzugsweise befinden sich in der Reaktionslösung 20 eine Mehrzahl von Lokalheizelementen 12 und eine Mehrzahl von Primernukleinsäuren 16, wovon vorzugsweise manche als Vorwärtsprimer und andere als Rückwärtsprimer für die PCR ausgebildet sind, wobei jedoch vorzugsweise sowohl die Vorwärtsprimer-Primernukleinsäuren als auch die Rückwärtsprimer-Primernukleinsäuren mit einem Verbindungsanschnitt 16a ausgebildet sind, welcher an die Verbindungsnukleinsäuren 14 binden kann.

Gemäß einer bevorzugten Ausführungsform sind die Lokalheizelemente 12 jeweils als ein Nanopartikel 12a ausgebildet und mit Verbindungsnukleinsäuren 14 funktionalisiert. Als eine Sorte von Lokalheizelementen 12 werden dabei Lokalheizelemente 12 angesehen, welche insbesondere hinsichtlich der Nanopartikel 12a und hinsichtlich der daran funktionalisierten Verbindungsnukleinsäuren 14 gleichartig sind. Die Verbindungsnukleinsäuren 14 sind (z.B. über eine 3'-Thiol-Bindung) derart an der Partikeloberfläche befestigt, dass das 5'-Ende der jeweiligen Verbindungsnukleinsäuren 14 vom Nanopartikel 12a abgewandt ist. Die Verbindungsnukleinsäuren 14 selbst dienen dabei vorzugsweise nicht als Primer für die Vervielfältigung der Nukleinsäuren bzw. für die PCR. Vielmehr sind die Nanopartikel 12a derart multifunktional einsetzbar, dass gegebenenfalls verschiedenartige Primernukleinsäuren 16 an die Verbindungsnukleinsäuren 14 binden können.

Da die optothermische Erhitzung der Nanopartikel 12a, d.h. das Erhitzen der Nanopartikel 12a mittels optischer Anregung, zur Erreichung oder zum Überschreiten der Denaturierungstemperatur für das Amplikon während dem Vervielfältigungsverfahren nur lokal stattfindet und nicht die gesamte Reaktionslösung 20 auf die Denaturierungstemperatur erhitzt werden muss, ist es erforderlich, dass das Amplikon zur Denaturierung über zumindest eine Primernukleinsäure 16 und eine Verbindungsnukleinsäure 14 an die Oberfläche der Nanopartikel gebunden werden.

Um die nachzuweisende Nukleinsäure 22 bzw. das Amplikon an die mit Verbindungsnukleinsäuren 14 funktionalisierten Nanopartikel 12a binden zu können, weisen mindestens einer der beiden für die PCR erforderlichen Primer-Sequenzen (vorzugsweise beide Primer-Sequenzen) zusätzlich zu dem Primerabschnitt 16b einen Verbindungsabschnitt 16a auf, wie etwa einen Überhang am 5'-Ende, der komplementär zu mindestens einem Teil der Verbindungsnukleinsäuren 14 auf den Nanopartikeln 12a ist. Das 3'-Ende der Primer-Sequenzen bzw. der Primernukleinsäuren 16 bzw. deren Primerabschnitte 16b wird dabei vorzugsweise freigehalten, so dass die Primernukleinsäuren 16 von dieser Seite beginnend durch die Polymerase elongiert werden können.

Vorzugsweise befinden sich zwischen dem Primerabschnitt 16b und dem Verbindungsabschnitt 16a einer jeweiligen Primernukleinsäure 16 eine oder mehrere abasische Modifikationen 28, um die Polymerase daran zu hindern, die Verbindungsabschnitte 16a zu überschreiben bzw. das Komplement zu den Verbindungsabschnitten 16a zu bilden. Dies ist insbesondere dann relevant, wenn die Primernukleinsäuren 16 nicht an eine Verbindungsnukleinsäure 14 gebunden sind, sondern sich separat von einer Verbindungsnukleinsäure 14 und einem Lokalheizelement 12 in der Lösung befinden. Die zumindest eine abasische Modifikation bietet somit den Vorteil, dass auch in den folgenden Zyklen der PCR die Verbindungsnukleinsäuren 14 und die Verbindungsabschnitte 16a der Primernukleinsäuren 16 einzelsträngig vorliegen, d.h. quasi als eine einzelsträngige Überhangsequenz, und somit das Amplikon bzw. die zu vervielfältigende Nukleinsäure 22 an die Verbindungsnukleinsäuren 14 auf den Nanopartikeln 12a binden kann und in der durch optothermisches Aufheizen lokal erhitzbaren Zone um die Nanopartikel 12a bzw. um die Nanopartikel-Oberfläche denaturiert werden können.

In Schritt b) findet ein Annealing zumindest eines Teils der Primernukleinsäuren 16 mit der einzelsträngig vorliegenden Nukleinsäure 22 statt, d.h. die Primernukleinsäuren 16 hybridisieren mit ihrem Primerabschnitt 16b an die zu vervielfältigende Nukleinsäure 22. Damit die Nukleinsäure 22 einzelsträngig vorliegt, kann ein einleitender, ggf. globaler, Erhitzungsschritt, in welchem beispielsweise die gesamte Reaktionslösung 20 auf oder über die Denaturierungstemperatur erhitzt wird, vorteilhaft sein. Dieser einleitende Erhitzungsschritt kann beispielsweise vor Schritt a) und/oder zwischen Schritt a) und b) stattfinden.

In Schritt c) erfolgt ein Elongieren der in der Reaktionslösung 20 befindlichen Nukleinsäuren 22, welche mit Primernukleinsäuren 16 gebunden sind. Insbesondere erfolgt dabei eine enzymatische Reaktion, bei welcher mittels einer Polymerase der an die Nukleinsäure 22 gebundene Primerabschnitt 16b der Primernukleinsäure 16 zu einem Amplikon 22a elongiert wird und auf diese Weise die Nukleinsäure 22 zum Doppelstrang komplettiert wird. Dabei wird bevorzugt der Verbindungsabschnitt 16a der Primernukleinsäure 16 von der Polymerase nicht erfasst bzw. zum Doppelstrang komplettiert, was insbesondere durch ein Bereitstellen der zumindest einen abasischen Modifikation 28 zwischen dem Verbindungsabschnitt 16a und dem Primerabschnitt 16b erreicht werden kann.

In Schritt d) erfolgt ein Binden der mit der elongierten Primernukleinsäure 16 hybridisierten Nukleinsäure 22 an eine der Verbindungsnukleinsäuren 14 an dem Lokalheizelement 12. Dadurch wird die nun doppelsträngig vorliegende Nukleinsäure 22 bzw. das Amplikon 22a zumindest teilweise, vorzugsweise jedoch vollständig, in die Umgebung 29 des Lokalheizelements 12 gebracht, welche mittels lokalem Heizen auf und/oder über die Denaturierungstemperatur erhitzt werden kann.

In Schritt e) wird das zumindest eine Lokalheizelement 12 erhitzt, was beispielsweise in dem Fall, dass das Lokalheizelement 12 als ein Nanopartikel 12a ausgebildet ist, mittels einer optischen Anregung 26, beispielsweise durch Laser-Strahlung, erfolgen kann. Dabei wird das Lokalheizelement 12 sowie eine Umgebung 29 des Lokalheizelements 12 auf und/oder über die Denaturierungstemperatur erhitzt, so dass sich die in der Umgebung 29 des Lokalheizelements 12 befindliche doppelsträngigen Nukleinsäuren, d.h. die Nukleinsäure 22 bzw. die Amplikons 22a und/oder die Primernukleinsäuren 16 und/oder die Verbindungsnukleinsäuren 14, zumindest teilweise, vorzugsweise jedoch vollständig voneinander trennen und zumindest teilweise, vorzugsweise jedoch vollständig, wieder als einzelsträngige Nukleinsäuren vorliegen.

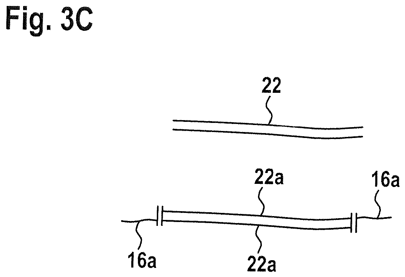

Mit den Schritten a) bis e) wurde demnach ein Amplikon 22a erzeugt, wodurch ein Komplementär, d.h. eine Nukleinsäure mit einer zur Nukleinsäure 22 zumindest teilweise komplementären Nukleotidsequenz, zur Nukleinsäure 22 erzeugt wurde. Durch zumindest eine Wiederholung kann die Nukleinsäure 22 somit kopiert bzw. vervielfältigt werden. In Figur 3C sind beispielhaft eine doppelsträngige, zu vervielfältigende Nukleinsäure 22 und zwei zu einem Doppelstrang hybridisierte Amplikons 22a dargestellt. Der Doppelstrang aus den Amplikons 22a weist dabei an jedem der beiden Enden einen von den Primernukleinsäuren 16 stammenden Verbindungsabschnitt 16a auf, mittels welchen der Amplikon-Doppelstrang an ein oder zwei Lokalheizelemente 12 bzw. deren Verbindungsnukleinsäuren 14 binden kann.

Die Schritte a) bis e) können dabei in einer Vielzahl von Zyklen durchlaufen werden, beispielsweise zwischen 20 und 1000 Zyklen, insbesondere mehr als 300 Zyklen, um dadurch eine exponentielle Vervielfältigung der zu Beginn vorliegenden Nukleinsäure 22 zu erzielen. Die Reihenfolge der Schritte b), c) und d) ist nicht an die dargestellte, beispielhafte Reihenfolge gebunden, sondern kann auch anders gewählt und/oder variiert werden. Der Zyklus kann insbesondere so oft durchlaufen werden, bis das gewünschte Ausmaß der Vervielfältigung erreicht ist. Die Anzahl der Durchläufe des Zyklus der Polymerase-Kettenreaktion ist vorzugsweise größer als 45, besonders vorzugsweise größer als 60, besonders vorzugsweise größer als 80, besonders vorzugsweise größer als 100, besonders vorzugsweise größer als 150, besonders vorzugsweise größer als 200. Mit einer großen Anzahl von Durchläufen kann vorteilhafterweise eine besonders hohe Vervielfältigung erreicht werden.

Die Anzahl der Durchläufe des Zyklus der Polymerase-Kettenreaktion ist vorzugsweise kleiner als 1000, besonders vorzugsweise kleiner als 750, besonders vorzugsweise kleiner als 500. Mit einer nicht zu hohen Zahl von Durchläufen des Zyklus kann vorteilhafterweise die Dauer der Vervielfältigung verringert werden. Außerdem können vorteilhafterweise negative Einflüsse von Verunreinigungen oder des Verbrauchs oder der Beschädigung von Reaktionspartnern, wie beispielsweise einer bei dem Verfahren eingesetzten Polymeralse, gering gehalten werden

Beim Denaturieren bzw. beim optothermischen, lokalen Heizen der Nanopartikel 12a wird unter Umständen nicht nur der Amplikon-Doppelstrang der zu vervielfältigenden Nukleinsäure 22 denaturiert, d.h. die Hybridisierung zwischen der elongierten Primernukleinsäure 16 und der Nukleinsäure 22 aufgebrochen, sondern gegebenenfalls auch Doppelstränge aus einer Verbindungsnukleinsäure 14 und einem daran gebundenen Verbindungsabschnitt 16a einer Primernukleinsäure 16. Dabei können sowohl elongierte Primernukleinsäuren 16, also Amplikons 22a, als auch noch nicht elongierte Primernukleinsäuren 16 wieder von den Verbindungsnukleinsäuren 14 an den Nanopartikeln 12a getrennt werden. Hierdurch können in folgenden Zyklen der PCR immer wieder neue Primernukleinsäuren 16 an die Verbindungsnukleinsäuren 14 an den Nanopartikeln 12a binden und es kann vorzugsweise zu einem ständigen Austausch von Primernukleinsäuren 16 an den Verbindungsnukleinsäuren 14 an den Nanopartikeln 12a kommen. Somit kann erreicht werden, dass Primernukleinsäuren 16 im Überschuss zu der Reaktionslösung 20 zugegeben werden können, also mehr Primernukleinsäuren 16 mit jeweils einem Verbindungsabschnitt 16a in der Reaktionslösung 20 vorhanden sein können, als gleichzeitig an die Verbindungsnukleinsäuren 14 an den Nanopartikeln 12a binden können. Dies kann beispielsweise vorteilhaft sein, um etwa die Dynamik der Amplifikationsreaktion zu beschleunigen.

Auch kann vorzugsweise hierdurch erreicht werden, dass die eigentliche Elongation der Primernukleinsäuren 16 durch die Polymerase nicht zwingend in der Nähe der Nanopartikel 12a, insbesondere über die Verbindungsnukleinsäuren 14 gebunden an die Nanopartikel 12a, stattfinden muss, wo unter Umständen ganz andere Ionen- und Ladungsverteilungen herrschen können, als in anderen Teilvolumina der Reaktionslösung in größerer Entfernung von den Nanopartikeln 12a. Denn der Prozess, in welchem eine Primernukleinsäure 16 ein Amplikon 22a oder eine zu vervielfältigende Nukleinsäure 22 findet und durch die Polymerase elongiert wird, kann gegebenenfalls auch weit entfernt von den Lokalheizelementen 12 bzw. Nanopartikeln 12a bzw. der Nanopartikeloberfläche stattfinden und anschließend kann dieses so geschaffene Amplikon mit mindestens einem überstehenden Verbindungsabschnitt 16a an die Verbindungsnukleinsäuren 14 an den Nanopartikeln 12a binden.

Gemäß einer weiteren bevorzugten Ausführungsform befinden sich in der Reaktionslösung 20 zumindest zwei verschiedene Arten bzw. Sorten von Primernukleinsäuren 16, wovon manche als Vorwärtsprimer und andere als Rückwärtsprimer ausgebildet sind, welche jedoch hinsichtlich ihres Verbindungsabschnitt 16a übereinstimmen und welche somit prinzipiell dazu geeignet sind, an die gleichen Verbindungsnukleinsäuren 14 zu binden.

In einer weiteren bevorzugten Ausführungsform befinden sich in der Reaktionslösung 20 erste Primernukleinsäuren 16, welche als Vorwärtsprimer ausgebildet sind und eine erste Nukleotidsequenz in ihrem Verbindungsabschnitt 16a aufweisen, und zweite Primernukleinsäuren 16, welche als Rückwärtsprimer ausgebildet sind, und eine von der ersten Nukleotidsequenz verschiedene zweite Nukleotidsequenz in ihrem Verbindungsabschnitt 16a aufweisen. Weiter befinden sich gemäß dieser bevorzugten Ausführungsform vorzugsweise entweder eine Nanopartikelsorte, die zwei verschiedenartige Verbindungsnukleinsäuren 14 aufweisen, wobei eine erste Verbindungsnukleinsäure 14 komplementär zur ersten Nukleotidsequenz und eine zweite Verbindungsnukleinsäure 14 komplementär zur zweiten Nukleotidsequenz ist, oder vorzugsweise zwei verschiedene Arten von Nanopartikeln 12a, die jeweils nur eine Art von Verbindungsnukleinsäuren 14 aufweisen, die entweder die zur ersten Nukleotidsequenz oder zur zweiten Nukleotidsequenz zumindest teilweise komplementär sind.

In einer weiteren bevorzugten Ausführungsform befinden sich in der Reaktionslösung 20 mindestens zwei unterschiedliche Kombinationen von Vorwärtsprimer- und Rückwärtsprimer- Primernukleinsäuren 16, die sich zwar hinsichtlich ihrer Primerabschnitte 16b unterscheiden, aber im Wesentlichen gleiche Verbindungsabschnitte 16a aufweisen. Eine Kombination von Vorwärtsprimer- und Rückwärtsprimer-Primernukleinsäuren 16, d.h. ein Primerpaar, ist dabei dadurch definiert, dass es gemeinsam ein doppelsträngiges Amplikon 22a erzeugen und/oder vervielfältigen kann.

In einer anderen Ausführungsform befinden sich in der Reaktionslösung 20 mindestens zwei unterschiedliche Kombinationen von Vorwärtsprimer- und Rückwärtsprimer-Primernukleinsäuren 16, die zumindest teilweise voneinander verschiedene Verbindungsabschnitte 16a aufweisen, und entsprechend viele verschiedene Arten von Lokalheizelementen 12, die jeweils die zu den unterschiedlichen Verbindungsabschnitten 16a komplementären Verbindungsnukleinsäuren 14 tragen. In einer weiteren bevorzugten Ausführungsform befinden sich in der Reaktionslösung mehrere Primerpaare, welche sich untereinander hinsichtlich Ihrer Verbindungsabschnitte und/oder hinsichtlich ihrer Primerabschnitte unterscheiden.

In einer weiteren bevorzugten Ausführungsform befindet sich in der Reaktionslösung 20 ein Primerpaar, bei dem nur entweder der Vorwärtsprimer oder der Rückwärtsprimer mit einem Verbindungsabschnitt 16a versehen ist, während der andere Primer keinen Verbindungsabschnitt 16a aufweist. Gemäß einer weiteren bevorzugten Ausführungsform können zusätzlich dazu Primerpaare in der Reaktionslösung bereitgestellt werden, bei welchen weder der Vorwärtsprimer noch der Rückwärtsprimer einen Verbindungsabschnitt 16a aufweist.

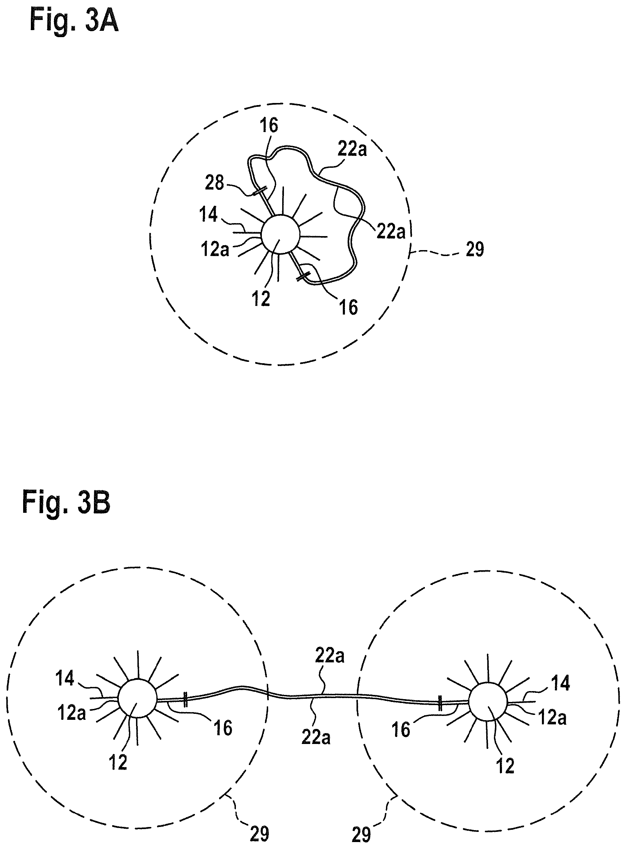

Gemäß manchen bevorzugten Ausführungsformen kann es dazu kommen, dass das Amplikon 22a bzw. die zu vervielfältigende Nukleinsäure 22 mit zwei Primernukleinsäuren 16 an ein Lokalheizelement 12 bzw. an ein Nanopartikel 12a gebunden wird, z.B. wenn sowohl Vorwärtsprimer Primernukleinsäure 16 und Rückwärtsprimer Primernukleinsäure 16 mit dem gleichen Verbindungsabschnitt 16a ausgestattet sind, oder beide Verbindungsabschnitte 16a (die von Vorwärtsprimer Primernukleinsäure 16 und Rückwärtsprimer Primernukleinsäure 16 mit unterschiedlichen Verbindungsabschnitten 16) auf einer Art von Lokalheizelementen 12 bzw. Nanopartikeln 12a binden können, die etwa mit beiden, entsprechenden Verbindungsnukleinsäuren 14 funktionalisiert sind, wie beispielhaft in Figur 3A dargestellt. Dabei ist ein Amplikon 22a als Doppelstrang aus einem elongierten Primerpaar, insbesondere aus einer elongierten Vorwärtsprimer-Primernukleinsäure 16 und einer elongierte Rückwärtsprimer-Primernukleinsäure 16, über zwei Verbindungsnukleinsäuren 14 mit dem selben Lokalheizelement 12 verbunden. Dies führt vorzugsweise dazu, dass sich die Amplikons zumindest teilweise, bevorzugt vollständig, innerhalb der Umgebung 29 des Lokalheizelements 12 befinden, welche von dem Lokalheizelement 12 auf und/oder über die Denaturierungstemperatur geheizt werden kann.

Hierdurch kann es ferner dazu kommen, dass das Amplikon 22a bzw. die zu vervielfältigende Nukleinsäure 22 beispielsweise besser und/oder homogener und/oder bei niedrigerer Anregungs-Leistungsdichte, beispielsweise bei niedrigerer Laser-Anregungsdichte falls die Lokalheizelemente 12 optisch mittels Laser-Strahlung angeregt werden, dehybridisiert werden können, da das Amplikon 22a im Durchschnitt über seine Länge näher an das Lokalheizelement 12 bzw. die Nanopartikeloberfläche gebracht wird als in einem Fall, wenn es nur mit einer Primernukleinsäure 16 oder nur an einem Ende an ein Lokalheizelement 12 bzw. ein Nanopartikel 12a gebunden ist, da die Erwärmung der Umgebung 29 mit zunehmender Entfernung von der Partikeloberfläche abnimmt.

Gemäß manchen bevorzugten Ausführungsformen kann es auch dazu kommen, dass das Amplikon mit zwei Primernukleinsäuren 16 zwischen zwei Lokalheizelemente 12 bzw. zwischen zwei Nanopartikel 12a gebunden wird, z.B. wenn sowohl Vorwärtsprimer Primernukleinsäure 16 und Rückwärtsprimer Primernukleinsäure 16 mit entsprechenden Verbindungsabschnitten versehen sind, wie in Figur 3B beispielhaft dargestellt. Hierdurch kann es ebenfalls dazu kommen, dass das Amplikon 22a beispielsweise besser und/oder homogener und/oder bei niedrigerer Anregungs-Leistungsdichte dehybridisiert werden kann, da das Amplikon 22a bzw. die Amplikons 22a von beiden Seiten geheizt wird, im Gegensatz zum Heizen von nur einer Seite, welches in dem Fall vorliegt, wenn die Amplikons 22a mit einer Primernukleinsäure 16 nur an einem Ende an ein Lokalheizelement 12 bzw. an nur ein Nanopartikel 12a gebunden sind.

Vorzugsweise werden die Primernukleinsäuren 16 und die mit Verbindungsnukleinsäuren 14 funktionalisierten Lokalheizelemente 12 gleichzeitig oder zeitlich nacheinander zur Reaktionslösung 20 für die Amplifikationsreaktion hinzugefügt.

In einer weiteren Ausführungsform werden die Primernukleinsäuren 16 zunächst auf die Verbindungsnukleinsäuren 14 an den Lokalheizelementen 12 hybridisiert und anschließend derart an den Verbindungsnukleinsäuren 14 immobilisiert, dass sich die Primernukleinsäuren 16 selbst während des Denaturierungsschrittes nicht mehr von der jeweiligen Verbindungsnukleinsäure 14 bzw. vom Lokalheizelement 12 lösen können. Diese Immobilisierung kann z.B. dadurch erfolgen, dass Primerabschnitte 16b der Primernukleinsäuren 16 und/oder die Verbindungsnukleinsäuren 14 Modifikationen aufweisen (wie z.B. mindestens eine Amino-Gruppe), die dann mittels einer chemischen Reaktion z.B. mit d-Linker (BS3- (bis(sulfosuccinimidyl)suberat)) ausreichend fest verbunden werden, so dass deren Bindung auch bei Temperaturen die gleich oder höher als die Denaturierungstemperatur sind, erhalten bleibt. Alternativ oder zusätzlich kann die Immobilisierung mittels Klick-Chemie erfolgen, beispielsweise mittels zumindest einer Azidmodifikation und einer Alkinmodifikation. Optional kann ein überschüssiges, verbindendes Reagenz für die chemische Reaktion zur Immobilisierung und/oder überschüssige nicht gebundene Primernukleinsäuren 16 vor der Amplifikationsreaktion, z.B. durch Waschen und/oder Aufreinigen und/oder Filtern und/oder Zentrifugieren, aus der Reaktionslösung 20 entfernt werden.

Wenngleich manche der soeben dargestellten, bevorzugten Ausführungsformen ausschließlich oder hauptsächlich als Ausführungsformen erläutert wurden, bei welchen die Lokalheizelemente 12 als Nanopartikel 12a ausgebildet sind, versteht sich von selbst, dass diese ebenso als bevorzugte Ausführungsformen der Erfindung gelten, wenn diese zusätzlich oder alternativ mit Mikroheizelementen 12b als Lokalheizelemente 12 realisiert werden.

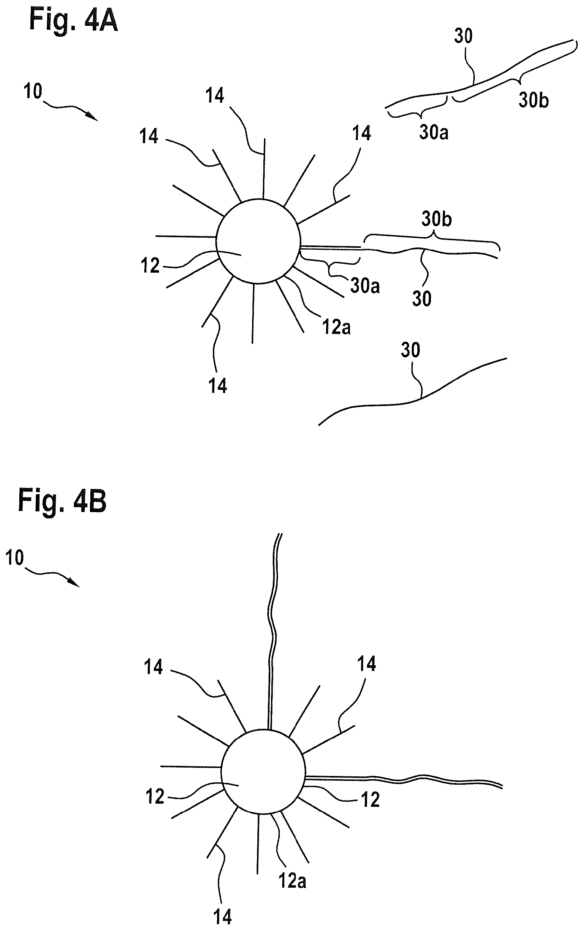

Im Folgenden wird eine zweite bevorzugte Ausführungsform erläutert, welche in den Figuren 4A bis 4D beispielhaft dargestellt ist. Diese weicht von der ersten bevorzugten Ausführungsform insbesondere dadurch ab, dass alternativ oder zusätzlich zu den Primernukleinsäuren 16 Primerkomplementärnukleinsäuren 30 in der Reaktionslösung 20 bereitgestellt werden. Wie in Figur 4A dargestellt, weisen die Primerkomplementärnukleinsäuren 30 einen Verbindungsabschnitt 30a und einen Primerkomplementärabschnitt 30b auf. Der Verbindungsabschnitt 30a dient entsprechend dem Verbindungsabschnitt 16a der Primernukleinsäuren 16 dazu, dass die Primerkomplementärnukleinsäuren 30 an die Verbindungsnukleinsäuren 14 bzw. die Lokalheizelemente 12 binden können.

Wie in Figur 4B dargestellt, kann, wenn eine Primerkomplementärnukleinsäure 30 an eine Verbindungsnukleinsäure 14 gebunden ist, mittels einer enzymatischen Reaktion, beispielsweise von einer Polymerase, die Verbindungsnukleinsäure 14 um eine Primer-Nukleotidsequenz bzw. einen Primerabschnitt elongiert werden, welche zu dem Primerkomplementärabschnitt 30b zumindest teilweise, bevorzugt vollständig, komplementär ist. Somit kann beispielsweise eine universelle Verbindungsnukleinsäure 14, welche keine Primereigenschaften aufweist, mithilfe einer Primerkomplementärnukleinsäure 30 mit einer Primer-Funktionalität versehen werden und/oder zu einer Primernukleinsäure bzw. zu einem Primer funktionalisiert bzw. elongiert werden. Durch ein globales Aufheizen der Reaktionslösung 20 und/oder durch ein lokales Heizen der Lokalheizelemente 12 können anschließend die Primerkomplementärnukleinsäuren 30 von den elongierten Verbindungsnukleinsäuren 14 getrennt werden, sodass die elongierten Verbindungsnukleinsäuren 14 einzelsträngig vorliegen und/oder als Primer für eine Amplifikationsreaktion verwendet werden können. In der in den Figuren 4A und 4B gezeigten Ausführungsform sind die Lokalheizelemente 12 als Nanopartikel 12a ausgebildet.

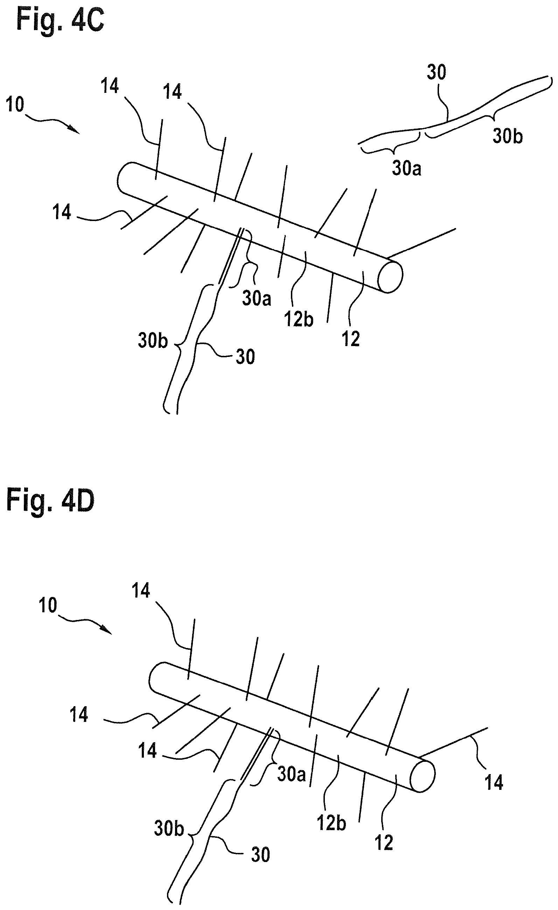

Die Figuren 4C und 4D zeigen eine Abwandlung der zweiten Ausführungsform aus den Figuren 4A und 4B, bei welcher die Lokalheizelemente 12 als Mikroheizelemente 12b, insbesondere als Mikroheizdrähte, ausgebildet sind.

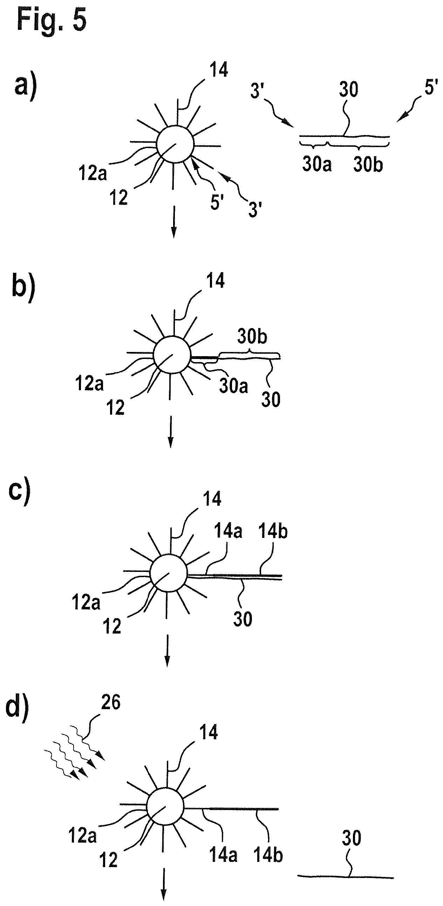

In Figur 5 ist das Individualisieren bzw. Funktionalisieren von mit Verbindungsnukleinsäuren 14 funktionalisierten Lokalheizelementen 12 beispielhaft dargestellt. Wenngleich die dargestellten und erläuterten Schritte anhand einer Ausführungsform dargelegt sind, in welcher die Lokalheizelemente 12 als Nanopartikel 12a ausgebildet sind, versteht sich von selbst, dass dies auch auf andere Ausführungsformen Anwendung findet, in welchen zusätzlich oder alternativ Lokalheizelemente 12 als Mikroheizelemente 12b ausgebildet sind.

In Schritt a) werden in der Reaktionslösung 20 mit Verbindungsnukleinsäuren 14 funktionalisierte Lokalheizelemente 12 und separat davon Primerkomplementärnukleinsäuren 30 bereitgestellt. Vorzugsweise erstreckt sich dabei in einem ersten Teil der Primerkomplementärnukleinsäure 30 beginnen mit dem 3'-Ende ein Verbindungsabschnitt 30a und darauf folgend ein Primerkomplementärabschnitt 30b, welcher sich vorzugsweise bis zum 5'-Ende der Primerkomplementärnukleinsäure 30 erstreckt. Vorzugsweise sind ferner die Verbindungsnukleinsäuren 14 mit ihrem 5'-Ende an dem Lokalheizelement 12 bzw. an dem Nanopartikel 12a bzw. an der Nanopartikeloberfläche befestigt, sodass die Verbindungsnukleinsäuren 14 und die Verbindungsabschnitte 30a in geeigneter Orientierung zueinander vorliegen, um ein Hybridisieren zu ermöglichen.

In Schritt b) erfolgt ein Annealing bzw. eine Hybridisierung, bei welcher die Verbindungsnukleinsäure 30 an eine der Verbindungsnukleinsäuren 14 bindet, und daraufhin mit dem Lokalheizelement 12 bzw. Nanopartikel 12a verbunden ist.

In Schritt c) erfolgt eine Elongation der Verbindungsnukleinsäure 14, welche mit der Primerkomplementärnukleinsäure 30 gebunden ist. Die Elongation erfolgt vorzugsweise durch eine chemische Reaktion, bevorzugt mittels eines Enzyms, wie etwa einer Polymerase. Dabei wird die Verbindungsnukleinsäure 14 derart elongiert, dass der elongierte Teil komplementär zu dem Primerkomplementärabschnitt 30b der Primerkomplementärnukleinsäure 30 ist und folglich zumindest teilweise, besonders bevorzugt jedoch vollständig, eine Nukleotidsequenz aufweist, welche zur Vervielfältigung der Nukleinsäure 22 als Primer verwendet werden kann. Die ursprüngliche Verbindungsnukleinsäure 14 kann dabei weiterhin als Verbindungsabschnitt 14a der elongierten Verbindungsnukleinsäure 14 dienen.

In Schritt d) erfolgt eine Denaturierung, sodass sich die Primerkomplementärnukleinsäure 30 von der elongierten Verbindungsnukleinsäure 14 trennt und daraufhin die Primerkomplementärnukleinsäure 30 und die an die Lokalheizelemente 12 gebundene, elongierte Verbindungsnukleinsäure 14 jeweils einzelsträngig in der Reaktionslösung 20 vorliegen. Die Denaturierung kann beispielsweise durch ein globales Erhitzen der Reaktionslösung 20 auf oder über die Denaturierungstemperatur erfolgen, oder durch ein lokales Erhitzen der Lokalheizelement 12 und der Umgebung 29 der Lokalheizelemente auf oder über die Denaturierungstemperatur, beispielsweise mittels einem optischen Anregen der als Nanopartikel 12a ausgebildeten Lokalheizelemente 12, während eine Temperatur der Reaktionslösung außerhalb der lokal geheizten Umgebung 29 im wesentlichen bzw. nahezu unverändert bleibt. Bevorzugt weist die Reaktionslösung außerhalb der lokal geheizten Umgebungen 29 der Lokalheizelemente 12 und bevorzugt auch innerhalb der Umgebungen 29 außerhalb der Denaturierungszeiten, in welchen die Lokalheizelemente 12 geheizt werden, eine Temperatur auf, welche im Wesentlichen eine Elongationstemperatur und/oder einer Annealingtemperatur entspricht.

Die mit den Schritten a) bis d) mit einer Primer-Funktionalität individualisierten bzw. funktionalisierten Lokalheizelemente 12, können anschließend als Primer verwendet werden, insbesondere im Rahmen einer Laser-PCR, wie diese beispielsweise aus der DE 10 2012 201 475 A bekannt ist.

Gemäß der zweiten bevorzugten Ausführungsform erfolgt somit für die Vervielfältigung der Nukleinsäure zunächst eine Erzeugung von Primern durch eine enzymatische Elongation von Verbindungsnukleinsäuren 14, welche an Lokalheizelemente 12 funktionalisiert sind. Dazu befinden sich in der Reaktionslösung 20 mindestens eine Sorte bzw. Art von Lokalheizelementen 12, wie etwa Nanopartikel 12a, die mit Verbindungsnukleinsäuren 14 funktionalisiert sind. Dabei sind die Verbindungsnukleinsäuren 14 derart (z.B. über eine 5'-Thiol-Bindung) an der Nanopartikeloberfläche bzw. an dem Lokalheizelement 12 befestigt, dass das 3'-Ende vom Nanopartikel 12a bzw. vom Lokalheizelement 12 abgewandt ist. Die Verbindungsnukleinsäuren 14 dienen ohne weitere Elongation vorzugsweise nicht als Primer-Sequenz. Diese Sorte bzw. Art von Nanopartikeln 12a bzw. Lokalheizelementen 12, d.h. die Lokalheizelemente 12 bzw. Nanopartikel 12a, welche mit universellen Verbindungsnukleinsäuren 14 funktionalisiert sind, sind somit multifunktional einsetzbar und nicht an die Vervielfältigung einer bestimmten, spezifischen Nukleinsäure 22 zweckgebunden.

Vielmehr werden die Lokalheizelemente 12 erst durch eine enzymatische Reaktion zu spezifisch funktionalisierten Lokalheizelementen 12. Hierfür werden die universell einsetzbaren Lokalheizelemente 12, welche mit universellen Verbindungsnukleinsäuren 14 funktionalisiert sind, in der Reaktionslösung 20 mit Primerkomplementärnukleinsäuren 30 gemischt, die mit anderen Worten ausgedrückt als Primervorlagen dienen. Die Primerkomplementärnukleinsäuren 30 bestehen aus zwei Teilsequenzen. Von 5' nach 3' gelesen kommt zuerst der Primerkomplementärabschnitt 30b, der zumindest teilweise, vorzugsweise jedoch vollständig, komplementär zur Primer-Sequenz ist, die am 3'-Ende der Verbindungsnukleinsäuren 14 erzeugt werden soll. Dann kommt der Verbindungsabschnitt 30a, der zumindest teilweise komplementär zu den Verbindungsnukleinsäuren 14 auf den Lokalheizelementen 12 ist.

Wenn eine Primerkomplementärnukleinsäure 30 mit einer Verbindungsnukleinsäure 14 am Lokalheizelement hybridisiert, kann ein in der Reaktionslösung 20 befindliches Enzym, wie etwa eine DNA-Polymerase, mit Hilfe der ebenfalls vorhandenen dNTPs (Desoxyribonukleosidtriphosphate) die Verbindungsnukleinsäure 14 am 3'-Ende verlängern. Dadurch entsteht ein an das Lokalheizelement 12 gebundenes Oligonukleotid das von 5' nach 3' einen Verbindungsabschnitt 14a, der im Wesentlichen der ursprünglichen Verbindungsnukleinsäure 14 entspricht, und einem gewünschten Primerabschnitt 14b, welcher die gewünschte Primer-Funktionalität aufweist, umfasst. Der Verbindungsabschnitt 14a kann dabei vorzugsweise als ein Spacer bzw. Abstandshalter fungieren, der vorzugsweise bewirkt, dass sich der Primerabschnitt 14b vorzugsweise in einem größeren Abstand zur Oberfläche des Lokalheizelements 12 bzw. des Nanopartikels 12a befindet.

An den Primerabschnitt 14b kann sodann das Target bzw. die zu vervielfältigende Nukleinsäure 22 hybridisieren, so dass eine Amplifikation mittels der bekannten Laser-PCR durchgeführt werden kann, wie diese für bereits spezifisch funktionalisiert bereitgestellten Nanopartikel aus der DE 10 2012 201 475 A bekannt ist.

In einer weiteren bevorzugten Ausführungsform kann die Verbindungsnukleinsäure 14 zumindest eine abasische Modifikation 28 aufweisen, die ein komplettes oder teilweises Überschreiben der Verbindungsnukleinsäure 14 durch die Polymerase in späteren PCR-Zyklen verhindern. Diese abasische Modifikation kann beispielsweise um wenige Nukleotidbasen von dem 3'-Ende der Verbindungsnukleinsäure 14 beabstandet sein.

In einer weiteren bevorzugten Ausführungsform befinden sich in der Reaktionslösung 20 erste Primerkomplementärnukleinsäuren 30, welche als Vorwärtsprimervorlagen dienen, und zweite Primerkomplementärnukleinsäuren 30, welche als Rückwärtsprimervorlagen dienen, wobei die ersten und die zweiten Primerkomplementärnukleinsäuren 30 vorzugsweise jeweils mit dem gleichen Verbindungsabschnitt 30a versehen sind.

In einer weiteren bevorzugten Ausführungsform befinden sich in der Reaktionslösung 20 erste Primerkomplementärnukleinsäuren 30, welche als Vorwärtsprimervorlagen ausgebildet sind, die einen Verbindungsabschnitt 30a mit einer ersten Nukleotidsequenz aufweisen, und zweite Primerkomplementärnukleinsäuren 30, welche als Rückwärtsprimervorlagen ausgebildet sind und einen Verbindungsabschnitt 30a mit einer zweiten Nukleotidsequenz aufweisen. Weiter befinden sich in dieser Ausführungsform entweder eine Art bzw. Sorte von Lokalheizelementen 12, die zwei Arten von Verbindungsnukleinsäuren 14 (eine erste komplementär zu den ersten und eine zweite komplementär zu den zweiten Primerkomplementärnukleinsäuren 30) oder zumindest zwei Arten bzw. Sorten von Lokalheizelementen 12, die jeweils nur entweder eine zur ersten oder nur eine zur zweiten Primerkomplementärnukleinsäuren 30 komplementäre Verbindungsnukleinsäure 14 aufweisen. Darüber hinaus kann auch eine Kombination dieser Ausführungsformen vorteilhaft sein.

In einer weiteren bevorzugten Ausführungsform befinden sich in der Reaktionslösung 20 mindestens zwei Primervorlagenpaare, die zumindest teilweise die gleichen Nukleotidsequenzen in den Verbindungsabschnitten 30a aufweisen, deren Primerkomplementärabschnitte 30b jedoch spezifisch für eine Amplifikation verschiedener Nukleinsäuren 22 sind.

In einer weiteren bevorzugten Ausführungsform befinden sich in der Reaktionsflüssigkeit 20 mindestens zwei verschiedene Arten von Primervorlagenpaare, die sich hinsichtlich Ihrer Verbindungsabschnitte 30a unterscheiden und entsprechend viele Arten von verschieden funktionalisierten Lokalheizelementen 12, deren Verbindungsnukleinsäuren 14 zu einer Art von Primervorlagenpaar zumindest teilweise komplementär sind.

Bei Verwendung einer Polymerase, die am 3'-Ende A-Überhange generiert (z.B. Taq-Polymerase), sollte die Primersequenz vorzugsweise so gewählt werden, dass das erste Nukleotid, das an die Primersequenz anschließt, ein Adenin ist, sonst ist die Elongation ggf. gestört. Mit anderen Worten kann es vorteilhaft sein, bei Verwendung einer Polymerase, die am 3'-Ende A-Überhange generiert, die Auswahl der Primersequenz derart vorzunehmen, dass beim Elongieren des Primers ohnehin eine Adenin-Base folgen würde, um auf diese Weise den A-Überhang quasi vorweg zu kompensieren.

Um zu Beginn bzw. vor Beginn der Vervielfältigungsreaktion bzw. PCR eine möglichst vollständige Hybridisierung der Primerkomplementärnukleinsäuren 30 an die Verbindungsnukleinsäuren 14 zu erzielen, kann es vorteilhaft sein, die Lokalheizelemente 12 mit daran befestigten Verbindungsnukleinsäuren 14 und die Primerkomplementärnukleinsäuren 30 unter veränderten Bedingungen vorzuinkubieren, als die Bedingungen, die während der Vervielfältigungsreaktion herrschen bzw. vorteilhaft sind. Beispielsweise kann zum Inkubieren eine andere Salzkonzentration in der Reaktionslösung 20, beispielsweise eine höhere Salzkonzentration (z.B. 15-20 mM MgCl₂), und/oder eine andere Temperatur der Reaktionslösung 20, beispielsweise eine geringere Temperatur (z.B. zwischen 20 und 50°C), vorteilhaft sein. In dieser Ausführungsform kann es vorteilhaft sein, dass erst nach einer solchen Vorinkubation weitere Reagenzien für die PCR bzw. Laser-PCR und/oder das Target bzw. die zu amplifizierende Nukleinsäure 22 zugegeben werden und sodann die eigentliche Amplifikationsreaktion gestartet wird.

In einer weiteren bevorzugten Ausführungsform findet das Elongieren bzw. Individualisieren bzw. Funktionalisieren der an die Lokalheizelemente 12 funktionalisierten Verbindungsnukleinsäuren 14, d.h. eine Synthese der Lokalheizelement-gebundenen Primer, durch ein Enzym anhand der Primerkomplementärnukleinsäuren 30 in einer ersten Reaktion statt und die Amplifikationsreaktion, in der das Target und/oder das Amplikon 22a bzw. die zu vervielfältigende Nukleinsäure 22 vervielfältigt werden, in einer zeitlich darauffolgenden zweiten Reaktion statt. Die erste und die zweite Reaktion können hier z.B. unter unterschiedlichen chemischen und/oder thermischen Bedingungen und/oder unter Verwendung von unterschiedlichen Polymerasen und/oder Nukleosiden stattfinden, sie können aber auch entweder gleichzeitig und/oder parallel und/oder zeitlich nacheinander unter gleichen Bedingungen stattfinden. In der ersten Reaktion kann die Synthese der an die Lokalheizelemente 12 gebundenen Primer durch ein Enzym in einem Schritt oder in mehreren zyklisch wiederholten Schritten erfolgen, z.B. durch wiederholtes (globales oder lokales) Erhitzen bzw. Aufheizen, wodurch die am Lokalheizelement 12 durch Elongation der Verbindungsnukleinsäuren 14 komplementär zum Primerkomplementärabschnitt 30b der Primerkomplementärnukleinsäuren 30 entstandenen Doppelstränge immer wieder denaturiert werden und im folgenden Zyklus wieder als freie Primervorlagen zur Erzeugung neuer Primer an anderen Verbindungsnukleinsäuren 14 zur Verfügung stehen. Dies bietet den Vorteil, dass geringere Mengen an Primerkomplementärnukleinsäuren 30 ausreichend sein können. Dies kann beispielsweise vorteilhaft sein, da geringere Mengen an Primerkomplementärnukleinsäuren 30, d.h. Primervorlagen, eine anschließende Amplifikationsreaktion, in der ein Target und/oder ein Amplikon 22a bzw. die zu vervielfältigende Nukleinsäure 22 vervielfältigt werden, optional einen geringeren (negativen) Einfluss auf die Amplifikationsreaktion nehmen.

In einer weiteren bevorzugten Ausführungsform können überschüssige, nicht gebundene Primervorlagen bzw. Primerkomplementärnukleinsäuren 30 und/oder dehybridisierte Primervorlagen bzw. Primerkomplementärnukleinsäuren 30 und/oder weitere Reaktionspartner wie z.B. Enzyme (z.B. Polymerase) und/oder Nukleoside vor Beginn der zweiten Reaktion, d.h. vor der Amplifikation der Nukleinsäure 22, aus der Reaktionslösung 20 entfernt werden, beispielsweise durch Waschen und/oder Aufreinigen und/oder Filtern und/oder Zentrifugieren.

In einer weiteren bevorzugten Ausführungsform können die Primervorlagen bzw. Primerkomplementärnukleinsäuren 30 nach der ersten Reaktion, in der die Primer synthetisiert wurden, in einer zweiten Reaktion beispielsweise durch ein Enzym verdaut und/oder zerstört und/oder zerschnitten und/oder zerkleinert werden, so dass diese in einer dritten Reaktion, in der ein Target und/oder ein Amplikon 22a bzw. die Nukleinsäure 22 vervielfältigt werden, einen geringeren (negativen) Einfluss nehmen können. Beispielsweise können durch das Enzym Uracil-DNA Glycosylase (UDG) Primervorlagen zerkleinert werden, die das Nukleotid Uracil enthalten, da Uracil durch dieses Enzym gezielt hydrolysiert wird. Alternativ oder zusätzlich können beispielsweise durch das Enzym RNase H2 Primervorlagen zerkleinert werden, die RNA-Nukleotide enthalten, da diese durch das Enzym RNase H2 zerstört werden können. In einer weiteren Ausführungsform können Enzyme und/oder Primervorlagen vor Beginn der dritten Reaktion z.B. durch Waschen und/oder Aufreinigen und/oder Filtern und/oder Zentrifugieren aus der Reaktionslösung 20 entfernt werden.

Dabei kann es vorteilhaft sein, die Primerkomplementärnukleinsäure zumindest teilweise mit Uracil-Basen anstatt mit Thymin-Basen bereitzustellen, um nach einer Elongation der Verbindungsnukleinsäuren eine Zerkleinerung der Primerkomplementärnukleinsäure bzw. zumindest deren Primerkomplementärabschnitte mittels UDG vornehmen zu können. Dabei kann es besonders vorteilhaft sein, wenn für eine darauffolgende Vervielfältigung der Nukleinsäure bzw. PCR keine Uracil-Nukleoside bereitgestellt werden, um ein Zerschneiden bzw. Zersetzen bzw. Zerkleinern von anderen Nukleinsäuren als die Primerkomplementärnukleinsäuren durch das UDG zu vermeiden.

Wenn die Primervorlagen bzw. die Primerkomplementärnukleinsäuren 30 vor der Amplifikationsreaktion, in der ein Target und/oder ein Amplikon 22a bzw. die Nukleinsäure 22 vervielfältigt werden, nicht oder nicht vollständig entfernt werden, können diese beispielsweise mit dem Amplikon 22a in dem Sinne konkurrieren, dass sie in der PCR mit dem Target und/oder dem Amplikon 22a um die Bindung an die elongierte Verbindungsnukleinsäure 14 konkurrieren. Die Konzentration an Primervorlagen bzw. Primerkomplementärnukleinsäuren 30 in der Reaktionslösung 20 darf deshalb in einem solchen Fall gegebenenfalls nicht zu hoch gewählt werden, weil die Amplifikation sonst zumindest teilweise gehemmt werden könnte. Für manche bevorzugte Ausführungsformen können beispielsweise Konzentrationen der Primerkomplementärnukleinsäuren 30 von 5 - 10 nM geeignet sein.

Im Lauf der Zeit kann allerdings die Primervorlage bzw. die Primerkomplementärnukleinsäure 30 bei Bindung an den neu entstandenen, partikelgebundenen Amplikonstrang 22a durch die 5'-3'-Exonuklease-Aktivität der DNA-Polymerase allmählich abgebaut werden, wenn diese z.B. den Rückwärtsprimer nach dessen Bindung an den partikelgebundenen Amplikonstrang elongiert. In diesem Fall könnten gegebenenfalls Rückwärtsprimer und Vorwärtsprimerkomplementärnukleinsäuren 30 unter Umständen beide auf den gleichen bzw. selben Strang des Amplikons 22a binden. Wenn dann der Rückwärtsprimer an seinem 3'Ende nach Vorlage des Amplikons 22a elongiert wird, kann ggf. das Enzym irgendwann die Vorwärtsprimerkomplementärnukleinsäure 30 erreichen und kann dann gegebenenfalls, je nach Enzym, aufgrund der sog. 5'-3'Exonuklease Aktivität beispielsweise die Vorwärtsprimerkomplementärnukleinsäure 30 zersetzen und/oder vom Amplikon entfernen.

Die im Folgenden dargelegten Ausführungen gelten sowohl für die erste als auch für die zweite bevorzugte Ausführungsform gleichermaßen:
Vorzugsweise bestehen die Verbindungsnukleinsäuren 14 und/oder die Primernukleinsäuren 16 und/oder die Primerkomplementärnukleinsäuren 30 zumindest teilweise aus, jedoch bevorzugt vollständig, im Wesentlichen aus DNA, insbesondere aus Oligonukleotiden. Im Wesentlichen vollständig bedeutet dabei, dass diese Nukleinsäuren als aus DNA bestehend erachtet werden, ungeachtet dessen, ob diese optional abasische Modifikationen und/oder Linker-Elemente, wie etwa Thiollinker, aufweisen. Sowohl Verbindungsabschnitte 16a und 30a als auch Verbindungsnukleinsäuren 14 und die Primerabschnitte 16b und/oder die Primerkomplementärabschnitte 30b können aber auch z.B. aus RNA, PNA, LNA oder Vergleichbarem bestehen oder diese umfassen. Sie können auch Modifikationen enthalten.

Vorzugsweise umfassen die Nukleotidsequenzen der Verbindungsnukleinsäure 14 und /oder der Primernukleinsäure 16 und/oder der Primerkomplementärnukleinsäure 30, insbesondere deren Verbindungsabschnitte 14a, 16a bzw. 30a, repetitive Sequenzen, d.h. sie umfassen mehrfache Wiederholungen von kurzen Teilsequenzen wie z.B. zehnfacher Wiederholung der Teilsequenz TTTG oder CAAA. Verbindungsnukleinsäure 14 und Primerkomplementärnukleinsäure 30 können aber jeweils auch nur eine Nukleotid-Art umfassen, wie z.B. 30 Adenin-Basen oder 30 Thymin-Basen. Nukleotidsequenzen mit sehr hohem Anteil an Adenin- oder Thymin-Basen können weniger geeignet sein, da sie z.B. durch Poly-A Carrier-RNA (wie sie z.B. bei der Nukleinsäure-Aufbereitung vielfach zur Verbesserung der Ausbeute eingesetzt wird und häufig aus Multi-Adenin-Sequenzen besteht) unter Umständen gestört und/oder blockiert werden könnten. Die Nukleotidsequenz in der Verbindungsnukleinsäuren 14 und/oder Primernukleinsäuren 16 und/oder der Primerkomplementärnukleinsäuren 30 weisen vorzugsweise eine geeignete Länge auf, damit ihre Hybridisierung bei den gegebenen Hybridisierungsbedingungen ausreichend dynamisch und effizient ist. Insbesondere sind die jeweiligen Längen der Verbindungsnukleinsäuren 14 und/oder der Primernukleinsäuren 16 und/oder der Primerkomplementärnukleinsäure 30 geeignet gewählt, um ein gewünschtes Schmelzverhalten aufzuweisen.

Beispielsweise können die Primernukleinsäuren 16 und/oder die Verbindungsnukleinsäuren 14 und/oder die Primerkomplementärnukleinsäuren 30 derart ausgestaltet sein, dass eine Bindung eines Verbindungsabschnitts 30a und/oder eines Verbindungsabschnitts 16a mit einer Verbindungsnukleinsäure 14 und/oder eine Bindung eines Primerabschnitts 16b und/oder eines Primerabschnitts 14b mit der Nukleinsäure 22 und/oder einem Amplikon 22b und/oder einem Target eine ähnliche Schmelztemperatur aufweisen. Vorzugsweise unterscheiden sich die Schmelztemperaturen um nicht mehr als ±5°C, besonders bevorzugt nicht mehr als ±2°C.

Die Primernukleinsäuren 16 und/oder die Verbindungsnukleinsäuren 14 und/oder die Primerkomplementärnukleinsäuren 30 können derart ausgestaltet sein, dass eine Länge eines Verbindungsabschnitts 30a einer Primerkomplementärnukleinsäure 30 und/oder eine Länge eines Verbindungsabschnitts 16a einer Primernukleinsäure 16 eine gleiche Länge aufweist wie die Verbindungsnukleinsäure 14. Gemäß einer anderen bevorzugten Ausführungsform können die Primernukleinsäuren 16 und/oder die Verbindungsnukleinsäuren 14 und/oder die Primerkomplementärnukleinsäuren 30 derart ausgestaltet sein, dass eine Länge eines Verbindungsabschnitts 30a einer Primerkomplementärnukleinsäure 30 und/oder eine Länge eines Verbindungsabschnitts 16a einer Primernukleinsäure 16 eine von der Länge der Verbindungsnukleinsäure 14 verschiedene Länge aufweist. Dabei wird die Bindungslänge im Wesentlichen durch die kürzere der beiden Längen bestimmt, d.h. durch die kürzere Länge des Verbindungsabschnitts 16a bzw. 30a und der Verbindungsnukleinsäure 14. Dies bietet den Vorteil, dass für eine Variation der Bindungslänge lediglich eine der Komponenten ausgetauscht werden muss, d.h. entweder die Verbindungsnukleinsäure 14 oder die Primernukleinsäure 16 bzw. die Primernukleinsäure 30.

Besonders bevorzugt können, beispielsweise in einem Kit, Lokalheizelemente mit unterschiedlich langen Verbindungsnukleinsäuren bereitgestellt bzw. angeboten werden. Mittels einer geeigneten Wahl von Primernukleinsäuren 16 und/oder Primerkomplementärnukleinsäuren 30 mit Verbindungsabschnitten 16a bzw. 30a, welche länger sind als die längsten Verbindungsnukleinsäuren 14 kann somit in einer Versuchsreihe durch Ausprobieren der verschiedenen Kombinationen die gewünschte Schmelztemperatur bzw. ein gewünschtes Schmelzverhalten erreicht werden.

Im Weiteren befinden sich auf den Nanopartikeln nicht nur Verbindungsnukleinsäuren 14 sondern auch weitere Füllnukleinsäuren, insbesondere Fülloligonukleotide, die nicht als Verbindungsnukleinsäuren 14 dienen, sondern beispielsweise eine Absättigung der Lokalheizelementoberfläche und/oder der Nanopartikeloberfläche und/oder eine Stabilisierung der Lokalheizelemente 12 bzw. der Nanopartikel 12a und/oder eine bessere sterische Zugänglichkeit der Verbindungsnukleinsäuren 14 ermöglichen.

Im Weiteren weisen die Verbindungsnukleinsäuren 14 eine Verbindungsnukleotidsequenz und zusätzlich eine universelle Spacersequenz auf (zwischen Nanopartikel-Oberfläche und Verbindungsnukleotidsequenz bzw. Verbindungsabschnitt 14a), die die Verbindungsnukleinsäure 14 bzw. die Verbindungsnukleotidsequenz in gewünschtem Abstand zur Nanopartikeloberfläche bzw. zum Lokalheizelement 12 positioniert, was sterische Vorteile bei der Amplifikation bieten kann.

Die Temperatur in der Reaktionslösung außerhalb der beheizten Umgebungen der Lokalheizelemente kann während einer PCR bzw. Laser-PCR konstant gehalten werden oder aber während der Laser-PCR variiert werden.

Ein Nachweis bzw. eine Quantifizierung der in der Amplifikationsreaktion erzeugten Target-Kopien der Nukleinsäure 22 kann z.B. durch (quantitative) Realtime-PCR und/oder PCR und/oder Gelelektorphorese und/oder mittels farbstoffgelabelten Hybridisierungs-Sonden erfolgen. Alternativ oder zusätzlich kann z.B. auch die Hybridisierung zwischen Nanopartikeln 12a bzw. die Verbindung der Nanopartikel 12a durch Hybridisierung von weiteren Nanopartikel-gebundenen Oligonukleotiden oder Amplikons z.B. als Rotverschiebung und Verbreiterung der Plasmonenresonanz im Extinktionsspektrum nachgewiesen werden und/oder durch Messung einer Transmissionsänderung der Reaktionslösung 20 bei einer oder mehren Wellenlängen, z.B. nach optothermischer Anregung der Nanopartikel 12a und einer hieraus resultierenden Denaturierung der Partikel-verbindenden DNA.

Im Folgenden wird die Erfindung anhand diverser konkreter Beispiele erläutert, ohne dass die Erfindung jedoch auf diese Beispiele beschränkt ist. Die angegebenen Nukleotidsequenzen sind in einer Übersicht im Anhang dargestellt.

### Beispiel 1

Gold-Nanopartikel mit 60 nm Durchmesser (bezogen von BBI Solutions) wurden als Lokalheizelemente verwendet und nach der Methode von Hurst et.al (s. J. Hurst et al., Anal Chem., 78(24), 8313-8318, 2006) mit Oligonukleotiden als Verbindungsnukleinsäure funktionalisiert. Hierbei wurde das Oligonukleotid mit Sequenz 4 eingesetzt. Nach Funktionalisierung und 4 Waschschritten lagen die Nanopartikel mit 600 pM in einem PBS Puffer (10mM NaCl, 2,11 mM KH₂PO₄ (P8709 von Sigma), 2,89 mM K₂HPO₄ (P8584 von Sigma), 0,01% Tween-20, 1 mM EDTA-S) vor.

Die endgültige Laser-PCR-Reaktion (Probenvolumen: 40 µl je Reaktionsgefäß) enthielt folgende Reagenzien:

| | |
|---|---|
| MgCl₂ | 18 mM |
| Tween 20 | 0,1% |
| Apta Taq Genotyping Master (Roche) | 1x |
| Freier Vorwärtsprimer (Sequenz 1, ohne Verbindungsabschnitt) | 700 nM |
| Hydrolysesonde (TaqMan-Sonde) (Sequenz 2 mit FAM-TAMRA) | 200 nM |
| Rückwärtsprimervorlage (Sequenz 3) | 5 nM oder keine |
| Goldnanopartikel 60nm (mit Verbindungsnukleinsäure (Sequenz 4)) | 60 pM |

Außerdem wurden 400.000 oder 40.000 Kopien DNA-Target (extrahierte genomische DNA von MRSA) in 40µl Reaktionslösung gegeben. Zur Negativkontrolle wurde Wasser anstatt Target Nukleinsäure verwendet. Vorwärtsprimer und Rückwärtsprimervorlage sowie die TaqMan-Sonde wurden so gewählt, dass das Resistenzgen MecA amplifiziert und Detektiert werden kann, das zum Beispiel im Genom des Methicillin-resistenten Staphylococcus aureus (MRSA) vorkommt.

Die Reaktion erfolgte in zwei Schritten:
Zunächst wurde ein Teilansatz zur Hybridisierung der Verbindungsnukleinsäuren angesetzt. Die komplette Menge an mit Sequenz 4 funktionalisierten Goldpartikeln und Primerkomplementärnukleinsäuren bzw. Primervorlagen (Sequenz 3) für eine komplette 40µl Reaktion wurden in einem Volumen von 10µl in Gegenwart von 15mM MgCl₂ (effektive Konzentration in 10µl) in einem 200µl-PCR-Tube für 15 min bei 37°C inkubiert. In einem Parallelansatz wurde die Primervorlage bzw. Primerkomplementärnukleinsäuren (Sequenz 3) durch das entsprechende Volumen Wasser ersetzt und analog inkubiert.

Während der Inkubation wurden die restlichen Reagenzien als Mastermix zusammengemischt. MgCl₂ wurde nur anteilig ergänzt, weil ein Teil davon bereits in der Vorinkubation enthalten war. Zu den 10µl aus der Vorinkubation wurden jeweils 26µl Mastermix und 4 µl 10-fach konzentriertes Target Nukleinsäure bzw. Wasser für die Negativkontrolle gegeben und gemischt. Die 40 µl wurden in die Reaktionskammern der Probenplatte gefüllt und die Einfüllöffnungen mit PCR-Sealing Folie verschlossen. Die Probenkammern haben eine Länge von 6mm, eine Breite von 4mm und eine Tiefe von 1,5mm. Die Probenplatte wurde in einen Plattenhalter gelegt und der Halter in den Lasercycler eingelegt. Die Probenplatte wurde im Lasercycler eine Minute lang auf 69°C aufgewärmt, ehe die Laser-PCR gestartet wurde. Die Proben werden gemäß diesem Beispiel in jedem Zyklus mit einem Laser mit 532nm Wellenlänge mit einer Intensität von 5-15kW/mm² meanderförmig abgescannt, indem ein fokussierter Laserstrahl in zwei Dimensionen relativ zum Probenvolumen verfahren wird, so dass jedes Nanopartikel im Reaktionsvolumen mindestens einmal je Zyklus optothermisch angeregt wird. (Entlang der dritten Achse des Probenvolumens (d.h. in dessen Tiefe) reicht die Fokuslänge des Laserfokusses aus, um alle Partikel mit ausreichender Intensität zu bestrahlen.) Das zweidimensionale Abscannen wird mittels einer langsamen und einer schnellen Relativbewegung zwischen Laserfokus und Probe erreicht: Das langsame Verfahren entlang der 6mm Längs-Ausdehnung der Reaktionskammer erfolgt durch ein Verfahren der Probe mit einer Geschwindigkeit von 25mm pro Sekunde; gleichzeitig wird der Laserstrahl senkrecht zur Verfahrrichtung (d.h. die 4mm Breite der Reaktionskammer überstreichend) mit einem Galvanometer mit einer Zeilenfrequenz von 1428 Hz und einer Amplitude von ca. 5mm periodisch ausgelenkt, so dass sich hier in Querrichtung eine Verfahrgeschwindigkeit des Fokusses von ca. 7,1 m/s ergibt. Mit der typischen Fokusgröße von 15-20µm ergibt sich zusammen mit der Verfahrgeschwindigkeit des Fokusses somit eine Anregungsdauer je Nanopartikel von ca. 2-3µs.

Insgesamt wird in jedem Zyklus jedes Nanopartikel mindestens einmal optothermisch angeregt, wobei die Zyklen 400 Mal wiederholt werden. Die Zeitdauer zwischen zwei Scans pro Probe betrug 5 s. Die Temperatur wurde konstant bei 69°C gehalten.

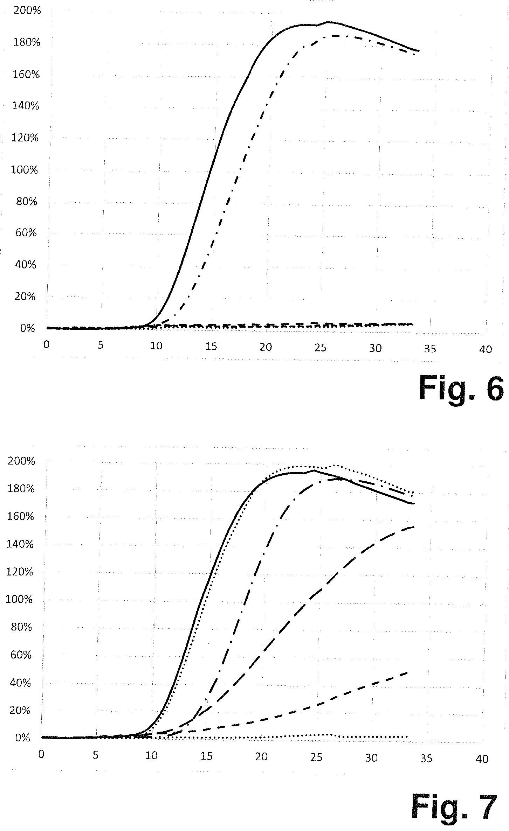

Das Ergebnis ist in Figur 6 dargestellt, in welcher in einem Graph die Änderung der Fluoreszenz bzw. das TaqMan-Signal, d.h. freigesetzte Fluoreszenz, da Farbstoff und Quencher der Hydrolyse-Sonde durch 5'-3' Exonuklease-Aktivität der Polymerase und somit durch eine Zersetzung der Hydrolyse-Sonde voneinander getrennt wurden, (vertikale Achse) gegen die Zeit in Minuten (horizontale Achse) aufgetragen ist. Die durchgezogene Linie entspricht dabei der Probe mit 400.000 Kopien des Targets und mit 5 nM Sequenz 3. Die Strichpunktlinie entspricht dabei der Probe mit 40.000 Kopien des Targets und mit 5 nM Primervorlage (Sequenz 3). Die lang-gestrichelte Line entspricht der Negativkontrolle nur mit 5 nM Sequenz 3. Die kurz-gestrichelte Linie entspricht 400.00 Kopien des Targets und ohne Sequenz 3 und die gepunktete Linie entspricht der Negativkontrolle ohne ID3. Dabei ist zu erkennen, dass lediglich die Reaktionslösungen, welche sowohl Kopien des Targets, als auch Sequenz 3 aufweisen eine Änderung der Fluoreszenz hervorrufen. Alle anderen Reaktionslösungen liefern im Wesentlichen kein Signal, welches sich von der Null-Linie deutlich unterscheiden würde.

In Gegenwart von 5 nM Primervorlage (Sequenz 3) zeigen 400.000 und 40.000 Kopien Target innerhalb von gut 10 min einen deutlichen Fluoreszenzanstieg und damit einen positiven Nachweis der Target-DNA. Ohne Primervorlage erhält man auch bei 400.000 Kopien Target im Ansatz keinen Signalanstieg. Die Ansätze ohne gDNA-Target sind ebenfalls negativ. Beides zeigt, dass es sich beim Signalanstieg in den Proben mit Target und Primervorlage (Sequenz 3) nicht um ein Artefakt handelt.

### Beispiel 2

Die Durchführung des Beispiels 2 entsprach im Wesentlichen der Durchführung des Beispiels 1, wobei abweichend davon die Primervorlage (Sequenz 3) mit 1 nM bis 20 nM eingesetzt bzw. weggelassen wurde. Es wurden jeweils 40.000 Kopien genomische DNA von MRSA eingesetzt. Das Ergebnis ist in dem Graphen in Figur 7 dargestellt, welcher analog zu Figur 6 eine Änderung der Fluoreszenz gegen die Zeitdauer in Minuten zeigt. Die untere gepunktete Linie entspricht der Reaktionslösung ohne Sequenz 3. Die Strichpunktlinie entspricht der Reaktionslösung mit 1 nM Sequenz 3. Die obere gepunktete Linie entspricht der Reaktionslösung mit 5 nM Sequenz 3. Die durchgezogene Linie entspricht der Reaktionslösung mit 10 nM Sequenz 3. Die lang-gestrichelte Line entspricht der Reaktionslösung mit 15 nM Sequenz 3. Die kurz-gestrichelte Linie entspricht der Reaktionslösung mit 20 nM Sequenz 3.

5 nM und 10 nM Primervorlage (Sequenz 3) liefern das beste Ergebnis. Das Signal steigt früh und steil an. Mit 1 nM Primervorlage (Sequenz 3) erhält man einen späteren, aber genauso steilen Signalanstieg. Die Amplifikation startet hier etwas verzögert, weil die Elongation der Verbindungsnukleinsäuren länger dauert, läuft aber genauso effektiv. Bei 15 nM und 20 nM Primervorlage steigt das Signal immer später und immer weniger steil an. Dies liegt daran, dass die Primervorlage bzw. die Primerkomplementärnukleinsäuren mit dem Target um die Bindung an den partikelgebundenen Primer konkurriert und somit die Amplifikation hemmen können.

### Beispiel 3

Für eine schnelle, effektive Elongation der Verbindungsnukleinsäuren wäre es prinzipiell wünschenswert, die Primervorlage in hoher Konzentration einzusetzen. Dies kann aber wie in Ausführungsbeispiel 2 zu sehen ist, zu einer Hemmung der Amplifikationsreaktion führen.

Eine Lösung hierfür kann die Zerstörung bzw. Zerkleinerung der Primervorlage bzw. Primerkomplementärnukleinsäure nach erfolgter Elongation der Verbindungsnukleinsäuren sein. Dies kann erreicht werden, indem man in die Primervorlage bzw. in die Primerkomplementärnukleinsäure über die gesamte Länge verteilt eines oder mehrere Ribonukleotide einbaut. Nach einem vorgeschalteten Schritt zur Elongation der Verbindungsnukleinsäuren kann die Primervorlage mit einer RNase enzymatisch zerstört werden, ehe die Laser-PCR gestartet wird.

Gold-Nanopartikel mit 60 nm Durchmesser (bezogen von BBI Solutions) wurden nach der Methode von Hurst et.al (s. J. Hurst et al., Anal Chem., 78(24), 8313-8318, 2006) mit Oligonukleotiden funktionalisiert. Hierbei wurde das Oligonukleotid mit Sequenz 4 eingesetzt. Nach Funktionalisierung und 4 Waschschritten lagen die Partikel 600 pM in einem PBS Puffer (10mM NaCl, 2,11 mM KH₂PO₄ (P8709 von Sigma), 2,89 mM K₂HPO₄ (P8584 von Sigma), 0,01% Tween-20, 1 mM EDTA-S) vor.

Die endgültige Laser-PCR-Reaktion (Probenvolumen: 40 µl pro Reaktionsgefäß) enthielt folgende Reagenzien:

| | |
|---|---|
| MgCl₂ | 18 mM |
| Tween 20 | 0,1% |
| Apta Taq Genotyping Master (Roche) | 1x |
| Freier Vorwärtsprimer (Oligo ID1) | 700 nM |
| Hydrolysesonde (Sequenz 2 mit FAM-TAMRA) | 200 nM |
| Ribonukleotid-haltige Rückwärtsprimervorlage (Sequenz 3-RN) | 60 nM |
| Goldnanopartikel 60nm (mit Verbindungsnukleinsäure mit Sequenz 4) | 60 pM |
| RNAse H2 (IDT) | 1 mU/µl oder keine |

Außerdem 400.000 oder 40.000 Kopien Target-DNA (genomische DNA von MRSA) in 40µl Probe, wobei zu Negativkontrolle Wasser anstatt Target-DNA verwendet wurde. Vorwärtsprimer und Rückwärtsprimervorlage sowie die TaqMan-Sonde wurden so gewählt, dass das Resistenzgen MecA amplifiziert und Detektiert werden kann, das zum Beispiel im Genom des Methicillin-resistenten Staphylococcus aureus (MRSA) vorkommt.

Die Reaktion erfolgte in drei Schritten:
Zunächst wurde ein Teilansatz zur vorgeschalteten Elongation der Verbindungsnukleinsäuren angesetzt.

Die komplette Menge an mit Sequenz 4 funktionalisierten Goldpartikeln und Primervorlage Sequenz 3-RN (mit Ribonukleotiden) für eine 40µl Reaktion wurden in einem Volumen von 10µl in Gegenwart von 15mM MgCl₂ und 1x Apta Taq Genotyping Master von Roche (jeweils effektive Konzentration in 10µl, wobei davon ausgegangen wird, dass ca. 3 mM MgCl₂ bereits im 1x Roche Apta Taq Genotyping Master enthalten sind) in einem 200 µl-PCR-Tube für 5 min bei 60°C i nkubiert.

Danach wurden 2µl thermophile RNase H2 aus Pyrococcus abysii (IDT) in der Konzentration von 20mU/µl zugegeben - verdünnt in speziellem Verdünnungspuffer von IDT (ergibt 1mU/µl in 40 µl Endvolumen). Die RNase H2 braucht für ihre Aktivität zusätzlich 0,01% Triton-X-100 (alternativ Tween-20). Der RNase H2-Verdünnungspuffer von IDT enthält 0,1 % Triton-X-100, sodass bei geeigneter Verdünnung des Enzyms auf diesem Wege ausreichend Triton-X-100 in die Reaktion gelangt. In der Laser-PCR-Reaktion selbst sind ohnehin 0,1% Tween-20.

Die RNase H2 aus Pyrococcus abysii spaltet DNA-RNA-Heteroduplizes am 5'-Ende von einzeln eingestreuten Ribonukleotiden. Diese sollten hierfür mindestens 8-10 Basen vom 5'-Ende bzw. mindestens 4 Basen vom 3'-Ende entfernt liegen.

Es wurde nochmals 5 min bei 60°C inkubiert, um die Primervorlage möglichst effektiv zu verdauen. Die Primervorlage Sequenz 3-RN enthält 5 Ribonukleotide gleichmäßig über die Sequenz verteilt, wird also in bis zu sechs Stücke zerlegt.

In einem parallelen Ansatz wurde statt RNAse H2 nur 2 µl RNAse-Verdünnungspuffer ohne Enzym zugegeben und analog inkubiert.

Während der Inkubation wurden die restlichen Reagenzien als Mastermix zusammengemischt. MgCl₂ und Apta Taq Genomic Master wurden nur anteilig ergänzt, weil ein Teil davon bereits in der Vorinkubation enthalten war. Zu den 12µl aus der Vorinkubation (inklusive RNAse- bzw. Puffer-Zugabe) wurden jeweils 24µl Mastermix und 4 µl 10-fach konzentriertes Target gegeben und gemischt. Die 40 µl wurden in die Reaktionskammern der Probenplatte wie in Beispiel 1 gefüllt und die Einfüllöffnungen mit PCR-Sealing Folie verschlossen. Die Prozessierung im Lasercycler erfolgte analog zu Beispiel 1.

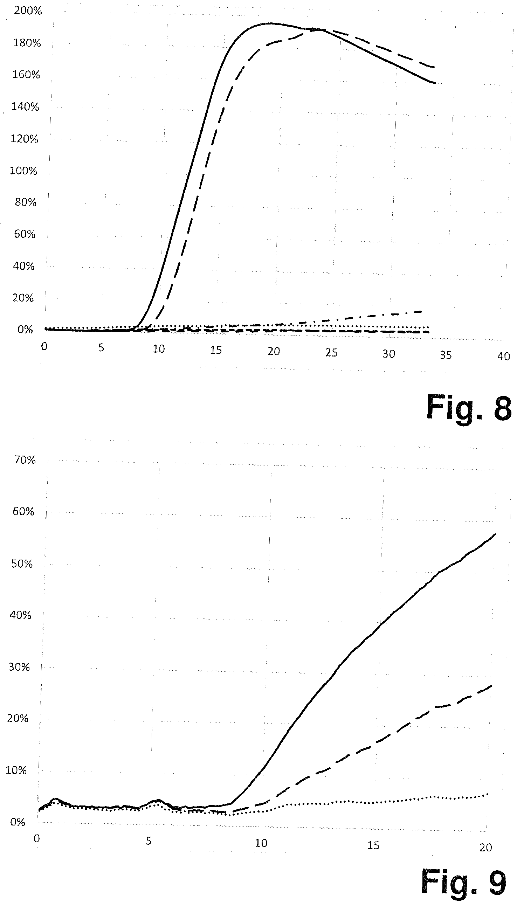

Das Ergebnis ist in dem Graphen in Figur 8 dargestellt, in welcher eine Änderung der Fluoreszenz gegen die Zeitdauer in Minuten zeigt. Die durchgezogene Linie entspricht der Reaktionslösung mit 400.000 Kopien der Taget-DNA mit RNAse. Die lang-gestrichelte Line entspricht der Reaktionslösung mit 40.000 Kopien mit RNAse. Die mittellang-gestrichelte Linie entspricht der Reaktionslösung mit Negativkontrolle mit RNAse. Die Strichpunktlinie entspricht der Reaktionslösung mit 400.000 Kopien der Target-DNA ohne RNAse. Die kurz-gestrichelte Linie entspricht der Reaktionslösung mit 40.000 Kopien der Target-DNA ohne RNAse. Die gepunktete Linie entspricht der Reaktionslösung mit Negativkontrolle ohne RNAse.

Die Primervorlage Sequenz 3-RN mit Ribonukleotiden kann bei Durchführung eines RNase-Verdauschrittes problemlos 60 nM eingesetzt werden. Bei 400.000 und 40.000 Kopien Target steigt das Signal jeweils einige Minuten früher an als im Format ohne RNase-Verdau beim besten Ergebnis im vorhergehenden Beispiel (Figur 7; 5 nM oder 10 nM Primervorlage Sequenz 3). Verzichtet man aber auf den RNAse-Verdau, so sind bereits 400.000 Kopien fast nicht mehr nachweisbar. Das Signal steigt erst sehr spät und sehr flach an. 40.000 Kopien sind völlig negativ. Dies deckt sich mit den Ergebnissen bei Sequenz 3-Konzentrationen größer 10 nM ohne Verdau, (Figur 7).

Große Mengen intakter Primervorlage bzw. Primerkomplementärnukleinsäuren hemmen die Amplifikation, weil die Primervorlage mit dem Target um die Bindung an den partikelgebundenen Primer konkurriert.

Im Format mit RNAse-Verdau kann die Primervorlage also in viel höherer Konzentration eingesetzt werden als im Format ohne RNAse-Verdau und man erreicht dadurch eine Beschleunigung der Amplifikation

### Beispiel 4

Gold-Nanopartikel mit 60 nm Durchmesser (bezogen von BBI Solutions) wurden nach der Methode von Hurst et.al (s. J. Hurst et al., Anal Chem., 78(24), 8313-8318, 2006) mit Oligonukleotiden funktionalisiert. Hierbei wurde das Oligonukleotid mit Sequenz 5 eingesetzt. Nach Funktionalisierung und 4 Waschschritten lagen die Partikel 600 pM in einem PBS Puffer (10mM NaCl, 2,11 mM KH₂PO₄ (P8709 von Sigma), 2,89 mM K₂HPO₄ (P8584 von Sigma), 0,01% Tween-20, 1 mM EDTA-S) vor.

Die endgültige Laser-PCR-Reaktion (Probenvolumen: 40 µl pro Reaktionsgefäß) enthielt folgende Reagenzien:

| | |
|---|---|
| MgCl₂ | 18 mM |
| Tween 20 | 0,1% |
| Apta Taq Genotyping Master (Roche) | 1x |
| Vorwärtsprimernukleinsäure (Sequenz 6) | 30 nM |
| Rückwärtsprimernukleinsäure (Sequenz 7) | 30 nM |
| Hydrolysesonde (Sequenz 2 mit FAM-TAMRA) | 200 nM |
| Goldnanopartikel 60nm (mit Verbindungsnukleinsäure mit Sequenz 5) | 60 pM |

Außerdem 400.000 oder 40.000 Kopien Target-DNA (genomische DNA von MRSA) in 40µl Probe, wobei zur Negativkontrolle Wasser anstatt Target-DNA verwendet wurde. Vorwärts- und Rückwärtsprimernukleinsäure sowie die TaqMan-Sonde wurden so gewählt, dass das Resistenzgen MecA amplifiziert und Detektiert wird, das zum Beispiel im Genom des Methicillin-resistenten Staphylococcus aureus (MRSA) vorkommt.

Die Nanopartikel-gebundene Verbindungsnukleinsäure mit Sequenz 5 ist in diesem Beispiel mit einem 3'-Thiol an den Nanopartikeln befestigt. Sowohl Vorwärtsprimernukleinsäure (Oligonukleotid mit Sequenz 6) als auch Rückwärtsprimernukleinsäure (Oligonukleotid mit Sequenz 7) tragen jeweils an dem dem 5'Ende zugewandten Teil einen Verbindungsabschnitt und zwischen Verbindungsabschnitt und Primerabschnitt je zwei Spacer9 Modifikationen als abasische Modifikationen.

Nachdem alle Komponenten gemischt wurden, wurden sie wie in den vorherigen Beispielen in die Probenplatten überführt und diese verschlossen. Anschließend wurde die Probenplatte zunächst für 1 Minute auf 88°C erh itzt, dann im Lasercycler eine Minute lang auf 69°C aufgewärmt, ehe die Laser-PCR gestartet wurde. Die Prozessierung im Lasercycler erfolgt analog zu Beispiel 1, jedoch abweichend hierzu mit einer Zeilenfrequenz von 800 Hz und 250 Zyklen.

Das Ergebnis ist in dem Graphen in Figur 9 dargestellt, welche eine Änderung der Fluoreszenz gegen die Zeitdauer in Minuten zeigt. Die durchgezogene Linie entspricht der Reaktionslösung mit 400.000 Kopien der Taget-DNA. Die lang-gestrichelte Line entspricht der Reaktionslösung mit 40.000 Kopien. Die gepunktete Linie entspricht der Reaktionslösung mit Negativkontrolle ohne Target-DNA.

In Gegenwart von 400.000 und 40.000 Kopien Target zeigt sich eine klare Fluoreszenzzunahme nach ca. 8 Minuten bzw. 10 Minuten und damit einen positiven Nachweis der Target-DNA. Ohne Target erhält man kaum Signalanstieg. Die Nanopartikel sind durch die Funktionalisierung mit Verbindungsnukleinsäuren mit Sequenz 5 zu universell einsetzbaren Lokalheizelementen geworden. Die an die Nanopartikel direkt gebundenen Verbindungsnukleinsäuren mit Sequenz 5 sind nicht als Primer für die nachzuweisende Nukleinsäure geeignet, allein schon deshalb, weil das für die Polymerase elongierbare 3'-Ende von Oligonukleotiden mit Sequenz 5 nicht frei zugänglich sondern an die Nanopartikel-Oberfläche gebunden ist. Durch den Einsatz von Vorwärtsprimernukleinsäure (Oligonukleotid mit Sequenz 6) und Rückwärtsprimernukleinsäure (Oligonukleotid mit Sequenz 7) jeweils mit Verbindungsabschnitt und abasischen Modifikationen können die universell einsetzbaren Lokalheizelemente dennoch für die spezifische Amplifikation und den spezifischen Nachweis der eingesetzten Nukleinsäure eingesetzt werden.

### Bezugszeichen

- 10: System
- 12: Lokalheizelement
- 12a: Nanopartikel
- 12b: Mikroheizelement
- 14: Verbindungsnukleinsäure
- 16: Primernukleinsäure
- 16a: Verbindungsabschnitt
- 16b: Primerabschnitt
- 18: Reaktionsgefäß
- 20: Reaktionslösung
- 22: Nukleinsäure
- 22a: Amplikon
- 24: (weiterer) Primer
- 26: optische Anregung
- 28: abasische Modifikation
- 29: Umgebung (eines Lokalheizelements)
- 30: Verbindungskomplementärnukleinsäure
- 30a: Verbindungsabschnitt
- 30b: Primerkomplementärabschnitt

### Anhang

Sequenzliste (Sequenz-Abfolge je von 5' nach 3'):
**Sequenz 1:** AGATGGTATGTGGAAGTTAGATTGG (SEQ ID No. 1)
**Sequenz 2:** 5'FAM-TCCTGGAATAATGACGCTATGATCCC-TAMRA (SEQ ID No. 2) FAM = 6-Carboxy-Fluorescein (Fluorezenzfarbstoff),
   TAMRA = 6-Carboxy-tetramethyl-rhodamin (Quencher)
**Sequenz 3:** GCAGAAAGACCAAAGCATACAT
   AAAAAAAAAAAAAAAAAAAAAAAAAAAAAAAAAAAAAAAA (SEQ ID No. 3)
**Sequenz 3-RN:** GCAGAAAGAcCAAAGCATAcAT
   AAAAAAAAaAAAAAAAAAAaAAAAAAAAAAaAAAAAAAAA (SEQ ID No. 4)
   Kleinbuchstaben geben Ribonukleotide an
**Sequenz 4:** 5'Thiol - TTTTTTTTTTTTTTTTTTTTTTTTTTTTTTTTTTTTTTTT (SEQ ID No. 5)
**Sequenz 5:**
   TTTTTTTTTTTTTTTTTTTTTTTTTTTTTTTTTTTTTTTT - 3'Thiol (C6) (SEQ ID No.6)
**Sequenz 6:** AAAAAAAAAAAAAAAAAAAAAAAAAAAAAAAAAAAAAAAA/Sp9//Sp9/ AGATGGTATGTGGAAGTTAGATTGG (SEQ ID No. 7; SEQ ID No 8)
**Sequenz 7:** AAAAAAAAAAAAAAAAAAAAAAAAAAAAAAAAAAAAAAAA/Sp9//Sp9/ ATGTATGCTTTGGTCTTTCTGC (SEQ ID No. 9; SEQ ID No 10)
   /Sp9/ ist eine abasische Modifikation "Spacer9"

## Patentansprüche

1. System (10) zum Vervielfältigen einer Nukleinsäure (22), umfassend:
- zumindest ein Lokalheizelement (12), welches mit zumindest einer Verbindungsnukleinsäure (14) funktionalisiert ist und dazu ausgelegt ist, mit einer Reaktionslösung (20) zur Durchführung einer Polymerase-Kettenreaktion zum Vervielfältigen einer Nukleinsäure (22) in Kontakt zu stehen, wobei das zumindest eine Lokalheizelement (12) als ein Nanopartikel (12a) ausgebildet ist und insbesondere dazu ausgelegt ist, durch eine Anregung Wärme an seine Umgebung (29) zu übertragen, oder wobei das zumindest eine Lokalheizelement (12) als ein Mikroheizelement (12b) ausgebildet ist und insbesondere dazu ausgelegt ist, durch ein resistives Heizen Wärme an seine Umgebung (29) zu übertragen, und
- zumindest eine Primernukleinsäure (16), welche dazu ausgelegt ist, an die zumindest eine Verbindungsnukleinsäure (14) zu binden und an die Nukleinsäure (22) zu binden, und/oder
- zumindest eine Primerkomplementärnukleinsäure (30), welche dazu ausgelegt ist, an die zumindest eine Verbindungsnukleinsäure (14) zu binden, und die Verbindungsnukleinsäure (14) mittels einer enzymatischen Reaktion um eine Primernukleotidsequenz zu elongieren.

2. System (10) nach Anspruch 1, wobei die Primerkomplementärnukleinsäure (30) zumindest einen Verbindungsabschnitt (30a) aufweist, welcher dazu ausgelegt ist, an die zumindest eine Verbindungsnukleinsäure (14) zu binden, und wobei die Primerkomplementärnukleinsäure (30) zumindest einen Primerkomplementärabschnitt (30b) aufweist, welcher dazu ausgelegt ist, die Verbindungsnukleinsäure (14) mittels einer enzymatischen Reaktion um eine Primernukleotidsequenz zu elongieren, und wobei eine Nukleotidsequenz in dem Verbindungsabschnitt (30a) der Primerkomplementärnukleinsäure (30) zumindest teilweise komplementär zu einer Nukleotidsequenz der Verbindungsnukleinsäure (14) ist und/oder wobei eine Nukleotidsequenz in dem Primerkomplementärabschnitt (30b) zumindest teilweise komplementär zu der Primernukleotidsequenz ist.

3. System (10) nach Anspruch 2, wobei die Primernukleinsäure (16) zwischen dem zumindest einen Verbindungsabschnitt (16a) und dem zumindest einen Primerabschnitt (16b) zumindest eine abasische Modifikation (28) aufweist und/oder wobei die Verbindungsnukleinsäure (14) eine abasische Modifikation (28) aufweist.

4. System (10) nach einem der vorhergehenden Ansprüche, umfassend eine Mehrzahl von Primernukleinsäuren (16), deren Primerabschnitte (16b) als Vorwärtsprimer und/oder als Rückwärtsprimer ausgebildet sind.

5. System (10) nach einem der vorhergehenden Ansprüche, wobei die zumindest eine Verbindungsnukleinsäure (14) und/oder die zumindest eine Primernukleinsäure (16) und/oder die zumindest eine Primerkomplementärnukleinsäure (30) jeweils zumindest teilweise als ein Oligonukleotid ausgebildet sind.

6. System (10) nach einem der vorhergehenden Ansprüche, wobei die Verbindungsnukleinsäure (14) und/oder die Primernukleinsäure (16) zumindest ein Immobilisierungselement aufweisen, welches dazu ausgelegt ist, die Primernukleinsäure (16), wenn an die Verbindungsnukleinsäure (14) gebunden, derart an der Verbindungsnukleinsäure (14) zu immobilisieren, dass die Primernukleinsäure (16) während eines Denaturierungsschrittes an die Verbindungsnukleinsäure (14) gebunden bleibt, wobei das zumindest eine Immobilisierungselement dazu ausgelegt ist, die Primernukleinsäure (16) mittels einer chemischen Reaktion an die Verbindungsnukleinsäure (14) zu immobilisieren.

7. Lokalheizelement (12) für eine Polymerase-Kettenreaktion zum Vervielfältigen einer Nukleinsäure (22), wobei das Lokalheizelement mit zumindest einer Verbindungsnukleinsäure (14) funktionalisiert ist und an die zumindest eine Verbindungsnukleinsäure (14) eine Primernukleinsäure (16) und/oder eine Primerkomplementärnukleinsäure (30) gebunden ist,
wobei die zumindest eine Primerkomplementärnukleinsäure (30) dazu ausgelegt ist, die Verbindungsnukleinsäure (14) mittels einer enzymatischen Reaktion um eine Primernukleotidsequenz zu elongieren, und wobei das zumindest eine Lokalheizelement (12) als ein Nanopartikel (12a) ausgebildet ist und insbesondere dazu ausgelegt ist, durch eine Anregung Wärme an seine Umgebung (29) zu übertragen, oder wobei das zumindest eine Lokalheizelement (12) als ein Mikroheizelement (12b) ausgebildet ist und insbesondere dazu ausgelegt ist, durch ein resistives Heizen Wärme an seine Umgebung (29) zu übertragen.

8. Verfahren zum Vervielfältigen einer Nukleinsäure (22) in einer Reaktionslösung (20), umfassend die Schritte:
- Bereitstellen von zumindest einem Lokalheizelement (12) in der Reaktionslösung (20), wobei das Lokalheizelement (12) mit zumindest einer Verbindungsnukleinsäure (14) funktionalisiert ist, wobei das zumindest eine Lokalheizelement (12) als ein Nanopartikel (12a) ausgebildet ist, welches durch eine Anregung Wärme an seine Umgebung (29) überträgt, wobei die Anregung als eine optische Anregung (26) erfolgt, oder wobei das zumindest eine Lokalheizelement (12) als ein Mikroheizelement (12b) ausgebildet ist, welches durch ein resistives Heizen Wärme an seine Umgebung (29) überträgt;
- Bereitstellen und/oder Erzeugen von zumindest einer Primernukleinsäure (16) in der Reaktionslösung (20), wobei die Primernukleinsäure (16) dazu ausgelegt ist, an die zumindest eine Verbindungsnukleinsäure (14) zu binden und an die Nukleinsäure (22) zu binden;
- Übertragen von Wärme durch das Lokalheizelement (12) an eine Umgebung (29) des Lokalheizelements (12) derart, dass eine über die zumindest eine Primernukleinsäure (16) und die zumindest eine Verbindungsnukleinsäure (14) mit dem zumindest einen Lokalheizelement (12) verbundene Nukleinsäure (22) auf und/oder über eine Denaturierungstemperatur erhitzt wird.

9. Verfahren nach Anspruch 8, wobei das Erzeugen der zumindest einen Primernukleinsäure (16) ein Bereitstellen einer Primerkomplementärnukleinsäure (30) in der Reaktionslösung (20) umfasst, wobei die Primerkomplementärnukleinsäure (30) einen Verbindungsabschnitt (30a) aufweist, welcher dazu ausgelegt ist, an die zumindest eine Verbindungsnukleinsäure (14) zu binden, und einen Primerkomplementärabschnitt (30b) mit einer Nukleotidsequenz aufweist, die zumindest teilweise komplementär zu der zu erzeugenden Primernukleinsäure (16) ist, und wobei das Verfahren nach dem Erzeugen der zumindest einen Primernukleinsäure (16) ferner den Schritt umfasst:
- Zerkleinern der zumindest einen Primerkomplementärnukleinsäure (30), wobei die Primerkomplementärnukleinsäure (30) zumindest teilweise mit Uracil-Basen bereitgestellt wird und besonders das Zerkleinern der zumindest einen Primerkomplementärnukleinsäure (30) zumindest teilweise durch ein Hydrolysieren der Uracil-Basen erfolgt.

10. Verfahren nach einem der Ansprüche 8 bis 9, ferner umfassend den Schritt:
- Immobilisieren der Primernukleinsäure (16) an der Verbindungsnukleinsäure (14) derart, dass die Primernukleinsäure (16) während eines Denaturierungsschrittes an die Verbindungsnukleinsäure (14) gebunden bleibt.

11. Verfahren nach einem der Ansprüche 8 bis 10, wobei das Vervielfältigen der Nukleinsäure (22) in der Reaktionslösung (20) mittels einer Polymerase-Kettenreaktion erfolgt.

## Claims

1. System (10) for amplifying a nucleic acid (22), comprising:
- at least one local heating element (12) which is functionalized with at least one linking nucleic acid (14) and is designed to be in contact with a reaction solution (20) for carrying out a polymerase chain reaction for amplifying a nucleic acid (22), wherein the at least one local heating element (12) is designed as a nanoparticle (12a) and is in particular designed to transfer heat to its surroundings (29) by excitation, or wherein the at least one local heating element (12) is designed as a microheating element (12b) and is in particular designed to transfer heat to its surroundings (29) by resistive heating, and
- at least one primer nucleic acid (16) which is designed to bind to the at least one linking nucleic acid (14) and to bind to the nucleic acid (22), and/or
- at least one primer complementary nucleic acid (30) which is designed to bind to the at least one linking nucleic acid (14) and to elongate the linking nucleic acid (14) by a primer nucleotide sequence by means of an enzymatic reaction.

2. System (10) according to claim 1, wherein the primer complementary nucleic acid (30) has at least one linking portion (30a) designed to bind to the at least one linking nucleic acid (14), and wherein the primer complementary nucleic acid (30) has at least one primer complementary portion (30b) which is designed to elongate the linking nucleic acid (14) by a primer nucleotide sequence by means of an enzymatic reaction, and wherein a nucleotide sequence in the linking portion (30a) of the primer complementary nucleic acid (30) is at least partially complementary to a nucleotide sequence of the linking nucleic acid (14) and/or wherein a nucleotide sequence in the primer complementary portion (30b) is at least partially complementary to the primer nucleotide sequence.

3. System (10) according to claim 2, wherein the primer nucleic acid (16) has at least one abasic modification (28) between the at least one linking portion (16a) and the at least one primer portion (16b) and/or wherein the linking nucleic acid (14) has an abasic modification (28).

4. System (10) according to any of the preceding claims, comprising a plurality of primer nucleic acids (16) of which the primer portions (16b) are designed as forward primers and/or as reverse primers.

5. System (10) according to any of the preceding claims, wherein the at least one linking nucleic acid (14) and/or the at least one primer nucleic acid (16) and/or the at least one primer complementary nucleic acid (30) are each at least partially formed as an oligonucleotide.

6. System (10) according to any of the preceding claims, wherein the linking nucleic acid (14) and/or the primer nucleic acid (16) comprise at least one immobilizing element which is designed to immobilize primer nucleic acid (16), when bound to the linking nucleic acid (14), on the linking nucleic acid (14) such that the primer nucleic acid (16) remains bound to the linking nucleic acid (14) during a denaturation step, wherein the at least one immobilizing element is designed to immobilize the primer nucleic acid (16) on the linking nucleic acid (14) by means of a chemical reaction.

7. Local heating element (12) for a polymerase chain reaction for amplifying a nucleic acid (22), wherein the local heating element is functionalized with at least one linking nucleic acid (14) and a primer nucleic acid (16) and/or a primer complementary nucleic acid (30) is bound to the at least one linking nucleic acid (14),
wherein the at least one primer complementary nucleic acid (30) is designed to elongate the linking nucleic acid (14) by a primer nucleotide sequence by means of an enzymatic reaction, and wherein the at least one local heating element (12) is designed as a nanoparticle (12a) and is in particular designed to transfer heat to its surroundings (29) by excitation, or wherein the at least one local heating element (12) is designed as a microheating element (12b) and is in particular designed to transfer heat to its surroundings (29) by resistive heating.

8. Method for amplifying a nucleic acid (22) in a reaction solution (20), comprising the steps of:
- providing at least one local heating element (12) in the reaction solution (20), wherein the local heating element (12) is functionalized with at least one linking nucleic acid (14), wherein the at least one local heating element (12) is designed as a nanoparticle (12a) which transfers heat to its surroundings (29) by excitation, wherein the excitation takes place as an optical excitation (26), or wherein the at least one local heating element (12) is designed as a microheating element (12b) which transfers heat to its surroundings (29) by resistive heating;
- providing and/or generating at least one primer nucleic acid (16) in the reaction solution (20), wherein the primer nucleic acid (16) is designed to bind to the at least one linking nucleic acid (14) and to bind to the nucleic acid (22);
- transferring heat via the local heating element (12) to the surroundings (29) of the local heating element (12) such that a nucleic acid (22) connected to the at least one local heating element (12) via the at least one primer nucleic acid (16) and the at least one linking nucleic acid (14) is heated to and/or above a denaturation temperature.

9. Method according to claim 8, wherein generating the at least one primer nucleic acid (16) comprises providing a primer complementary nucleic acid (30) in the reaction solution (20), wherein the primer complementary nucleic acid (30) has a linking portion (30a) which is designed to bind to the at least one linking nucleic acid (14) and a primer complementary portion (30b) having a nucleotide sequence that is at least partially complementary to the primer nucleic acid (16) to be generated, and wherein the method, after generating the at least one primer nucleic acid (16), further comprises the step of:
- comminuting the at least one primer complementary nucleic acid (30), wherein the primer complementary nucleic acid (30) is provided at least partially with uracil bases and particularly the comminution of the at least one primer complementary nucleic acid (30) is carried out at least partially by hydrolyzing the uracil bases.

10. Method according to one of claims 8 to 9, further comprising the step of:
- immobilizing the primer nucleic acid (16) on the linking nucleic acid (14) such that the primer nucleic acid (16) remains bound to the linking nucleic acid (14) during a denaturation step.

11. Method according to any of claims 8 to 10, wherein the amplification of the nucleic acid (22) in the reaction solution (20) is carried out by means of a polymerase chain reaction.

## Revendications

1. Système (10) pour l'amplification d'un acide nucléique (22), comprenant :
- au moins un élément de chauffage local (12) qui est fonctionnalisé avec au moins un acide nucléique de liaison (14) et qui est conçu pour être en contact avec une solution réactionnelle (20) pour la réalisation d'une réaction en chaîne par polymérase pour l'amplification d'un acide nucléique (22), dans lequel l'au moins un élément de chauffage local (12) est réalisé comme une nanoparticule (12a) et est en particulier conçu pour transférer de la chaleur à son environnement (29) au moyen d'une excitation, ou dans lequel l'au moins un élément de chauffage local (12) est réalisé comme un microélément de chauffage (12b) et est en particulier conçu pour transférer de la chaleur à son environnement (29) au moyen d'un chauffage résistif ; et
- au moins un acide nucléique amorce (16) qui est conçu pour se lier à l'au moins un acide nucléique de liaison (14) et pour se lier à l'acide nucléique (22), et/ou
- au moins un acide nucléique complémentaire amorce (30) qui est conçu pour se lier à l'au moins un acide nucléique de liaison (14) et pour rallonger l'acide nucléique de liaison (14) d'une séquence nucléotidique d'amorce au moyen d'une réaction enzymatique.

2. Système (10) selon la revendication 1, dans lequel l'acide nucléique complémentaire amorce (30) présente au moins une section de liaison (30a) qui est conçue pour se lier à l'au moins un acide nucléique de liaison (14), et dans lequel l'acide nucléique complémentaire amorce (30) présente au moins une section complémentaire d'amorce (30b) qui est conçue pour rallonger l'acide nucléique de liaison (14) d'une séquence nucléotidique d'amorce au moyen d'une réaction enzymatique, et dans lequel une séquence nucléotidique dans la section de liaison (30a) de l'acide nucléique complémentaire amorce (30) est au moins partiellement complémentaire d'une séquence nucléotidique de l'acide nucléique de liaison (14) et/ou dans lequel une séquence nucléotidique dans la section complémentaire d'amorce (30b) est au moins partiellement complémentaire de la séquence nucléotidique d'amorce.

3. Système (10) selon la revendication 2, dans lequel l'acide nucléique amorce (16) présente au moins une modification abasique (28) entre l'au moins une section de liaison (16a) et l'au moins une section d'amorce (16b) et/ou dans lequel l'acide nucléique de liaison (14) présente une modification abasique (28).

4. Système (10) selon l'une des revendications précédentes, comprenant une pluralité d'acides nucléiques amorces (16) dont les sections d'amorce (16b) sont conçues en tant qu'amorces directes et/ou amorces indirectes.

5. Système (10) selon l'une des revendications précédentes, dans lequel l'au moins un acide nucléique de liaison (14) et/ou l'au moins un acide nucléique amorce (16) et/ou l'au moins un acide nucléique complémentaire amorce (30) sont respectivement au moins partiellement réalisés en tant qu'oligonucléotide.

6. Système (10) selon l'une des revendications précédentes, dans lequel l'acide nucléique de liaison (14) et/ou l'acide nucléique amorce (16) présentent au moins un élément d'immobilisation qui est conçu pour immobiliser l'acide nucléique amorce (16), lorsqu'il est lié à l'acide nucléique de liaison (14), sur l'acide nucléique de liaison (14) de telle sorte que l'acide nucléique amorce (16) reste lié à l'acide nucléique de liaison (14) pendant une étape de dénaturation, dans lequel l'au moins un élément d'immobilisation est conçu pour immobiliser l'acide nucléique amorce (16) sur l'acide nucléique de liaison (14) au moyen d'une réaction chimique.

7. Élément de chauffage local (12) pour une réaction en chaîne par polymérase pour l'amplification d'un acide nucléique (22), dans lequel l'élément de chauffage local est fonctionnalisé avec au moins un acide nucléique de liaison (14) et un acide nucléique primaire (16) et/ou un acide nucléique complémentaire primaire (30) sont liés à l'au moins un acide nucléique de liaison (14),
dans lequel l'au moins un acide nucléique complémentaire amorce (30) est conçu pour rallonger l'acide nucléique de liaison (14) d'une séquence nucléotidique d'amorce au moyen d'une réaction enzymatique, et dans lequel l'au moins un élément de chauffage local (12) est réalisé comme une nanoparticule (12a) et est en particulier conçu pour transférer de la chaleur à son environnement (29) au moyen d'une excitation, ou dans lequel l'au moins un élément de chauffage local (12) est réalisé comme un microélément de chauffage (12b) et est en particulier conçu pour transférer de la chaleur à son environnement (29) au moyen d'un chauffage résistif.

8. Procédé pour l'amplification d'un acide nucléique (22) dans une solution réactionnelle (20), comprenant les étapes consistant à :
- mettre à disposition au moins un élément de chauffage local (12) dans la solution réactionnelle (20), dans lequel l'élément de chauffage local (12) est fonctionnalisé avec au moins un acide nucléique de liaison (14), dans lequel l'au moins un élément de chauffage local (12) est réalisé comme une nanoparticule (12a) qui transmet de la chaleur à son environnement (29) au moyen d'une excitation, dans lequel l'excitation est effectuée en tant qu'excitation optique (26), ou dans lequel l'au moins un élément de chauffage local (12) est réalisé en tant que microélément de chauffage (12b) qui transmet de la chaleur à son environnement (29) au moyen d'un chauffage résistif ;
- mettre à disposition et/ou produire au moins un acide nucléique amorce (16) dans la solution réactionnelle (20), dans lequel l'acide nucléique amorce (16) est conçu pour se lier à l'au moins un acide nucléique de liaison (14) et pour se lier à l'acide nucléique (22) ;
- transmettre de la chaleur par l'intermédiaire de l'élément de chauffage local (12) à un environnement (29) de l'élément de chauffage local (12) de telle sorte qu'un acide nucléique (22) lié à l'au moins un élément de chauffage local (12) par l'intermédiaire de l'au moins un acide nucléique amorce (16) et de l'au moins un acide nucléique de liaison (14) est chauffé à une température de dénaturation et/ou au-dessus de celle-ci.

9. Procédé selon la revendication 8, dans lequel la production de l'au moins un acide nucléique amorce (16) comprend une mise à disposition d'un acide nucléique complémentaire amorce (30) dans la solution réactionnelle (20), dans lequel l'acide nucléique complémentaire amorce (30) présente une section de liaison (30a) qui est conçue pour se lier à l'au moins un acide nucléique de liaison (14) et présente une section complémentaire d'amorce (30b) comportant une séquence nucléotidique qui est au moins partiellement complémentaire de l'acide nucléique amorce (16) à produire, et dans lequel le procédé comprend en outre, après la production de l'au moins un acide nucléique amorce (16), l'étape consistant à :
- fragmenter l'au moins un acide nucléique complémentaire amorce (30), dans lequel l'acide nucléique complémentaire amorce (30) est au moins partiellement mis à disposition avec des bases uracile et, en particulier, la fragmentation de l'au moins un acide nucléique complémentaire amorce (30) est au moins partiellement effectuée par une hydrolyse des bases uracile.

10. Procédé selon l'une des revendications 8 à 9, comprenant en outre l'étape consistant à :
- immobiliser l'acide nucléique amorce (16) sur l'acide nucléique de liaison (14) de telle sorte que l'acide nucléique amorce (16) reste lié à l'acide nucléique de liaison (14) pendant une étape de dénaturation.

11. Procédé selon l'une des revendications 8 à 10, dans lequel l'amplification de l'acide nucléique (22) dans la solution réactionnelle (20) est effectuée au moyen d'une réaction en chaîne par polymérase.
